# EUROPÄISCHE PATENTANMELDUNG

(11) **EP 4 497 815 A1**
(43) Veröffentlichungstag der Anmeldung: **29.01.2025**
(21) Anmeldenummer: 23187413.2
(22) Anmeldetag: 25.07.2023
(51) Int. Cl.: C12M 1/34, C12M 1/36

(54) **APPARATUR UND ZUGEHÖRIGES VERFAHREN ZUR ERMITTLUNG EINER OPTIMIERTEN ZUSAMMENSETZUNG EINES KULTURMEDIUMS**

(71) Anmelder: LABMaiTE GbmH, 79110 Freiburg (DE)
(72) Erfinder: Bermeitinger, Jonas, 79110 Freiburg (DE); Raith, Dennis, 79110 Freiburg (DE); Mertelsmann, Roland, 79104 Freiburg (DE); Melachrinos, Roman, 79110 Freiburg (DE); Sapre, Avani, 79110 Freiburg (DE); Stingl, Matias, 79100 Freiburg (DE); Lapierre, Frédéric, 79100 Freiburg (DE); Huber, Robert, 79110 Freiburg (DE)
(74) Vertreter: Mertzlufft-Paufler, Cornelius

(57) **Zusammenfassung**

Zur Beschleunigung der Ermittlung optimaler Bedingungen zur Kultivierung eines bestimmten biologischen Organismus (14) wird eine Apparatur (1) und ein zugehöriges Verfahren vorgeschlagen, mit dem sich, mithilfe eines Computers und autonom, also ohne jegliches menschliches Zutun, eine optimierte Zusammensetzung eines Kulturmediums (3) und gegebenenfalls zusätzliche optimierte Wirkfaktoren ermitteln lassen, die die Kultivierung des Organismus (14) begünstigen. Durch diesen Ansatz lässt sich in sehr kurzer Zeit die optimierte Zusammensetzung des Kulturmediums (3) für einen spezifischen biologischen Organismus (14) automatisiert ermitteln (Fig. 1) oder beispielsweise ein optimiertes Verhältnis von wenigstens zwei Organismen (14a, 14b), die gemeinsam kultiviert werden sollen. (Fig. 1)

## Beschreibung

Die Erfindung betrifft eine Apparatur zur computer-implementierten Ermittlung einer optimierten Zusammensetzung eines Kulturmediums und/oder eines optimierten Verhältnisses von (wenigstens) zwei Organismen. Hierfür umfasst die Apparatur eine Anzahl an N Reaktionsgefäßen, die räumlich voneinander getrennt sind, beispielsweise durch entsprechende Gefäßwandungen, und die mit N Zusammensetzungen des Kulturmediums befüllbar sind. Denn so kann im Rahmen einer Versuchsreihe bestehend aus N Kultivierungsversuchen, wobei in jedem der N Kultivierungsversuche jeweils (mindestens) ein biologischer Organismus (also insbesondere die erwähnten wenigstens zwei Organismen) in dem jeweiligen Reaktionsgefäß kultiviert wird, das Wachstum/die Kultivierung des zu untersuchenden Organismus (oder der wenigstens zwei gemeinsam zu untersuchenden Organismen) in der jeweiligen Zusammensetzung des Kulturmediums in dem jeweiligen Reaktionsgefäß untersucht werden. Hierbei wird von dem (jeweiligen) Organismus (oder von wenigstens zwei Organismen) in jedem der N Reaktionsgefäße ein jeweiliger biologischer Prozess ausgeführt, wobei sich diese Prozesse aufgrund der unterschiedlichen Zusammensetzungen des jeweiligen Kulturmediums und/oder eines unterschiedlichen Verhältnisses der wenigstens zwei Organismen in ihrer jeweiligen Ausprägung unterscheiden können. Die Optimierung dieser Prozesse und damit der Kultivierung des jeweiligen Organismus kann mittels der Erfindung dabei autonom und automatisiert erzielt werden.

Ferner umfasst die Apparatur einen Roboter, der zum Herstellen der jeweiligen Zusammensetzung des Kulturmediums in dem jeweiligen Reaktionsgefäß und/oder zur Herstellung des besagten Verhältnisses der wenigstens zwei Organismen eingerichtet ist. Mit anderen Worten kann die Apparatur somit die jeweiligen Zusammensetzungen und/oder das besagte Verhältnis im jeweiligen Reaktionsgefäß selbständig ohne jeglichen menschlichen Eingriff von außen herstellen. So kann nämlich die jeweilige Zusammensetzung und/oder das jeweilige Verhältnis im nachfolgenden jeweiligen Kultivierungsversuch untersucht und automatisiert von der Apparatur evaluiert werden. Mit dem Roboter kann also insbesondere alternativ oder ergänzend zur Anpassung/Herstellung der Zusammensetzung des Kulturmediums das Verhältnis der wenigstens zwei Organismen im jeweiligen Reaktionsgefäß eingestellt werden.

Ferner umfasst die Apparatur bevorzugt wenigstens eine Prozessmessvorrichtung, die zum Messen einer jeweiligen Prozessantwort oder eines jeweiligen Prozesszustands eingerichtet ist, die/der innerhalb des jeweiligen Reaktionsgefäß von dem Organismus während der Versuchsreihe erzeugt wird. Wie noch genauer erläutert werden wird, kann die Apparatur bevorzugt auch (wenigstens) einen separaten Inokulum-Bioreaktor umfassen, in welchem ein Inokulum (häufig als "Vorkultur", selten auch als "Inokulat" bezeichnet) des (jeweiligen) zu kultivierenden Organismus bereitgehalten werden kann oder bereitgehalten ist. Dieser Vorkultur-Reaktor stellt somit für den Roboter eine Vorkultur des (insbesondere jeweiligen) Organismus zur Verfügung, der in den Reaktionsgefäßen in unterschiedlichen jeweiligen Zusammensetzungen des Kulturmediums und/oder in unterschiedlichen Verhältnissen zusammen mit wenigstens einem zweiten Organismus kultiviert werden soll.

Die Erfindung betrifft ferner ein zugehöriges Verfahren zur computer-implementierten Ermittlung einer optimierten Zusammensetzung eines Kulturmediums (in welchem wenigstens ein Organismus kultiviert werden soll) und/oder zur computer-implementierten Ermittlung eines optimierten Verhältnisses von wenigstens zwei Organismen, die gemeinsam (in einem Kulturmedium) kultiviert werden sollen. Es sei bereits an dieser Stelle angemerkt, dass dieses Verfahren bevorzugt mittels einer erfindungsgemäßen Apparatur ausgeführt werden kann, wie sie hierin beansprucht und beschrieben ist. Beispielsweise die Herstellung des Kulturmediums muss aber nicht zwingend mit einem Roboter geschehen, etwa dann, wenn das Verfahren vornehmlich dazu eingesetzt wird, ein gewünschtes Verhältnis von zwei Organismen für ein bereits optimiertes (gleichbleibendes) Kulturmedium zu ermitteln.

Die Kultivierung von Mikroorganismen in einem Kulturmedium stellt in vielen Fällen eine Herausforderung für den Anwender dar, insbesondere dann, wenn mehrere Organismen, beispielsweise Co-Kulturen, in dem Kulturmedium gemeinsam kultiviert werden sollen. Soll ein bestimmter Modellorganismus genauer untersucht werden, müssen oftmals zunächst langwierige und aufwendige Vorversuche gestartet werden, um überhaupt ein geeignetes Nährmedium, oftmals als Kulturmedium bezeichnet, aufzufinden und dabei auch die Zusammensetzung der Bestandteile dieses Kulturmediums so lange zu verändern, bis der Modellorganismus (bzw. ggf. die Organismen) in dem Kulturmedium auch über längere Zeiträume wachsen oder einfach am Leben gehalten werden kann, wobei der Modellorganismus dabei auch ggf. einen Stoff produzieren und/oder verbrauchen/verstoffwechseln kann.

Ebenso ist es nicht trivial, bei Co-Kulturen ein geeignetes Verhältnis der beteiligten Organismen einzustellen, damit diese gemeinsam erfolgreich kultiviert werden können, selbst dann, wenn bereits ein geeignetes Kulturmedium bekannt ist. Aber auch hier kann gerade die Wahl der Zusammensetzung des Kulturmediums entscheidenden Einfluss auf die Kultivierung der Organismen nehmen.

Im Stand der Technik wurden bislang klassische Methoden wie beispielsweise "one-factor-at-a-time" eingesetzt, die es oftmals erlauben, kausale Zusammenhänge zwischen dem Modellorganismus und dem zugehörigen Kulturmedium zu ermitteln und anschließend auf Basis dieser Zusammenhänge ein Modell zu erstellen, welches die Optimierung des Kulturmediums erlaubt.

Daneben besteht ein weiterer klassischer Ansatz darin, statistische Versuchspläne, sogenannte Design-of-Experiments (DoE), durchzuführen, bei denen oftmals mehrere Wirkparameter gleichzeitig variiert werden, um so zügig und schnell eine optimierte Zusammensetzung eines Kulturmediums oder ein geeignetes Verhältnis von zwei Organismen zu finden. Bei diesem Ansatz können aber oftmals keine Kausalzusammenhänge mehr ermittelt werden, insbesondere wenn diese zu komplex sind.

Ausgehend von diesem vorbekannten Stand der Technik und den Vorveröffentlichungen WO 2020/016223 A1 und EP 4 039 791 A1 hat sich die Erfindung die Aufgabe gesetzt, einen neuen Ansatz zu entwickeln, mit dem der Aufwand und die Zeit, die zur Kultivierung von Organismen aufgewendet werden muss, reduziert werden kann.

Zur Lösung dieser Aufgabe sind erfindungsgemäß bei einer Apparatur die Merkmale von Anspruch 1 vorgesehen. Insbesondere wird somit erfindungsgemäß zur Lösung der Aufgabe bei einer Apparatur der eingangs genannten Art vorgeschlagen, dass die Apparatur einen Controller aufweist, der dazu eingerichtet ist, die mittels der wenigstens einen Prozessmessvorrichtung während der jeweiligen Versuchsreihe gemessenen insgesamt N Prozessantworten und/oder N Prozesszustände jeweils Computer-implementiert anhand einer gemeinsamen Zielvorgabe, die für alle in den Reaktionsgefäßen parallel, vorzugsweise gleichzeitig, ablaufenden Prozesse gilt, zu bewerten. Ferner ist vorgesehen, dass der Controller dazu eingerichtet ist, auf Basis dieser N Bewertungen Computer-implementiert die jeweilige Zusammensetzung des Kulturmediums, welche in dem jeweiligen Reaktionsgefäß in einer nachfolgenden Versuchsreihe hergestellt werden soll, dem Roboter in Form einer Instruktion vorzugeben. Anschließend kann der Roboter dann zu Beginn der nachfolgenden neuen Versuchsreihe die vorgegebene Zusammensetzung des Kulturmediums im jeweiligen Reaktionsgefäß anlegen.

Ein solcher gemessener Prozesszustand kann beispielsweise eine bestimmte aktuelle Biomassekonzentration oder ein bestimmter ph-Wert oder eine sonstige messbare Zustandsgröße sein, die den momentanen Zustand des Organismus charakterisiert (beispielsweise am Ende eines Kultivierungsversuchs), der gerade im jeweiligen Reaktionsgefäß kultiviert wird. Eine gemessene Prozessantwort, etwa die Veränderung der Biomassekonzentration oder ein verändertes Spektrum, das auf einen von dem Organismus ausgeführten biologischen Prozess zurückzuführen ist, kann beispielsweise Rückschlüsse ermöglichen auf eine bestimmte Aktivität des Organismus, beispielsweise einen Stoffwechsel oder eine(n) bestimmte(n) Stoffproduktion oder Stoffverbrauch. So kann etwa die Apparatur autonom die Zusammensetzung des Kulturmediums im Hinblick auf eine gewünschten Rate einer Stoffproduktion oder eines Stoffverbrauchs (durch den Organismus) optimieren.

Alternativ oder aber ergänzend zu dem zuletzt genannten Merkmal kann vorgesehen sein, dass der Controller dazu eingerichtet ist, auf Basis der N Bewertungen computer-implementiert ein Verhältnis von wenigstens zwei unterschiedlichen Organismen (die in dem jeweiligen Reaktionsgefäß während eines Kultivierungsversuchs gemeinsam kultiviert werden), welches Verhältnis in dem jeweiligen Reaktionsgefäß in einer nachfolgenden Versuchsreihe hergestellt werden soll, dem Roboter in Form einer Instruktion vorzugeben. Mit dieser Instruktion wird also festgelegt, in welchem relativen Verhältnis die wenigstens zwei unterschiedlichen Organismen in der nachfolgenden Versuchsreihe erneut untersucht werden sollen. Hierbei kann zum Beispiel eine vorab bereits (insbesondere mit Hilfe des erfindungsgemäßen Verfahrens) optimierte Zusammensetzung des Kulturmediums beibehalten werden, oder diese Zusammensetzung wird erst später noch optimiert, oder aber diese wird gleichzeitig mit dem Verhältnis der Organismen von dem Controller optimiert. Anschließend kann der Roboter dann zu Beginn der nachfolgenden neuen Versuchsreihe das vorgegebene Verhältnis der wenigstens zwei Organismen im jeweiligen Reaktionsgefäß anlegen.

Somit kann mittels der Erfindung nicht nur eine Nährmedium-Optimierung, sondern z.B. auch eine Optimierung einer Induktion einer bestimmten Proteinexpression oder z.B. eine Optimierung von bestimmten Antibiotika-Kombinationen durchgeführt werden. Damit ergeben sich zahlreiche Anwendungsmöglichkeiten der Erfindung in unterschiedlichen Gebieten der Bioverfahrenstechnik, der Medizintechnik und in der pharmazeutischen Verfahrenstechnik.

Ein wesentlicher Vorteil der Erfindung besteht darin, dass es die erfindungsgemäße Apparatur ermöglicht, automatisiert und ohne menschliches Zutun (also autonom) eine optimale Zusammensetzung eines Kulturmediums zu ermitteln, und zwar für einen ganz bestimmten Organismus (menschliche oder tierische Zelle, Bakterium, Pilz etc) oder auch für eine bestimmte Kombination von wenigstens zwei Organismen. Dieses Konzept ist sowohl auf Bakterien aber auch auf sonstige Mikroorganismen anwendbar, sowie auf pflanzliche, tierische oder menschliche Zellen, die kultiviert werden sollen und ferner auch zum Beispiel auf Co-Kulturen. Hierbei könnten sich langfristig insbesondere Anwendungen für die Medizin ergeben, weil gerade die Kultivierung von Zellen für eine patienten-spezifische Therapie immer noch eine große Herausforderung darstellt, die mit dem erfindungsgemäßen Ansatz angegangen werden kann.

Die Erfindung erscheint auch für die Bioverfahrenstechnik von hohem Interesse, da mit Hilfe der Erfindung Kosten- und zeitoptimiert sehr schnell ermittelt werden kann, wie ein Kulturmedium optimal auf einen bestimmten biologischen Organismus abgestimmt werden kann.

Die erfindungsgemäße Apparatur (und das später noch genauer erläuterte zugehörige Verfahren) lässt sich aber nicht nur einsetzen, um einen einzigen Organismus zu untersuchen. Vielmehr kann gemäß einer möglichen Ausgestaltung vorgesehen sein, dass in einzelnen oder allen der N Reaktionsgefäße wenigstens zwei unterschiedliche biologische Organismen (insbesondere sogenannte Co-Kulturen oder synthetische mikrobielle Konsortien) während einer Versuchsreihe / während eines einzelnen Kultivierungsversuchs kultiviert werden. Hierbei kann der Controller dazu eingerichtet sein, vorzugsweise mit Hilfe des Roboters (oder aber auf sonstige Weise), ein Verhältnis (insbesondere ein Volumen- / Masse- oder Mischungsverhältnis) der wenigstens zwei unterschiedlichen biologischen Organismen in dem jeweiligen Reaktionsgefäß auf Basis der zuvor erläuterten N Bewertungen (die dann jeweils für einen Prozess ermittelt werden, der auf einzelne oder aber alle der wenigstens zwei Organismen zurückzuführen ist) computer-implementiert vorzugeben.

Hierbei ist es sogar möglich, dass der Controller in einer Versuchsreihe zunächst stets dieselbe Zusammensetzung des Kulturmediums einsetzt, um zunächst für diese Zusammensetzung ein optimales Verhältnis der wenigstens zwei unterschiedlichen Organismen zu ermitteln. Durch ein gezieltes Mischen der wenigstens zwei unterschiedlichen Organismen kann beispielsweise eine bestimmte Produktbildung (als einer möglichen Zielgröße/Zielvorgabe, die der Controller berücksichtigt), an welcher die Organismen beteiligt sind, vollautomatisiert mit der Apparatur optimiert werden. Bei Verwendung mehrerer unterschiedlicher Organismen erhöht sich zwar die Komplexität des Lösungsraums; der erfindungsgemäße Ansatz ist durch den Einsatz einer künstlichen Intelligenz aber anders als bei klassischen Methoden in der Lage, auch dann noch eine Optimierung zu erzielen.

Somit kann eine erfindungsgemäße Apparatur auch eingesetzt werden, um die Interaktion mehrerer Mikroorganismen in einem bestimmten (unter Umständen stets gleichbleibenden) Kulturmedium zu untersuchen. Hierbei kann der Controller, insbesondere auf Basis einer künstlichen Intelligenz, zusätzlich oder aber alternativ zur Zusammensetzung des Kulturmediums, das Verhältnis der wenigstens zwei unterschiedlichen biologischen Organismen in dem jeweiligen Reaktionsgefäß vorgeben. Werden z.B. drei, vier oder noch mehr unterschiedliche Organismen mit der Apparatur untersucht, kann der Controller dabei - ähnlich wie bei den unterschiedlichen Bestandteilen des Kulturmediums - auch einzelne der unterschiedlichen Organismen ganz weglassen. So kann also die Apparatur / die künstliche Intelligenz insbesondere eine Selektion vornehmen unter unterschiedlichen in der Apparatur bereit gehaltenen Organismen, um eine bestimmte Zielvorgabe zu erreichen.

Die Apparatur kann das Verhältnis der wenigstens zwei unterschiedlichen biologischen Organismen beispielsweise dadurch einstellen, dass zu Beginn einer Versuchsreihe ein bestimmtes Mengen- bzw. Konzentrationsverhältnis von wenigstens zwei unterschiedlichen Inokula (die jeweils einen oder mehrere der jeweiligen wenigstens zwei unterschiedlichen Organismen umfassen), im jeweiligen Reaktionsgefäß mit dem Roboter hergestellt wird. Dabei muss die Biomassekonzentration in jeweiligen vorgehaltenen Inokulum nicht zwingend angepasst werden; vielmehr können unterschiedliche Mengen des Inokulums in das jeweilige Reaktionsgefäß abgegeben werden und/oder es kann beispielsweise eine Probe des jeweiligen Inokulums entsprechend verdünnt werden, um so die Biomassekonzentration des Organismus in dieser Probe abzusenken.

Entsprechend kann die Apparatur auch wenigstens zwei Inokulum-Bioreaktoren (diese können in einem Gerät realisiert sein) aufweisen. Dadurch kann z.B. je ein Inokulum für jeden/einen/ mehrere der wenigstens zwei unterschiedlichen Organismen bereithaltbar sein oder bereitgehalten sein.

Die Verwendung von räumlich voneinander getrennten Reaktionsgefäßen hat den Vorteil, dass die biologischen Prozesse gleichzeitig aber räumlich getrennt im jeweiligen Reaktionsgefäß und damit unbeeinflusst voneinander ablaufen können, sodass unterschiedliche Zusammensetzungen des Kulturmediums und/oder unterschiedliche Verhältnisse der unterschiedlichen Organismen gleichzeitig von der Apparatur untersucht werden können (im Hinblick auf ein gewünschtes Prozessziel, beispielsweise ein starkes und/oder schnelles Wachstum des Organismus und/oder eine gewünschte Stoffproduktion/ Stoffverbrauch durch den Organismus, d.h. insbesondere einen gewünschten Stoffwechsel des Organismus). Durch diesen parallelen und automatisierten Ansatz wird eine enorme Beschleunigung der Untersuchung erzielt, sodass die Apparatur innerhalb weniger Tage bis Wochen die optimierte Zusammensetzung des Kulturmediums bestimmen kann und ggf. zusätzlich noch weitere optimierte Kulturbedingungen (wie optimierte Werte für zusätzliche einzustellende Wirkparameter wie etwa Temperatur des Reaktionsgefäßes, Bestrahlungsstärke, Zusammensetzung einer Gasatmosphäre in dem Reaktionsgefäß, etc.), oder beispielsweise ein optimiertes Verhältnis von wenigstens zwei unterschiedlichen Organismen, die in einer bestimmten Zusammensetzung des Kulturmediums interagieren sollen (gemäß der Zielvorgabe).

Das Kulturmedium, das beispielsweise in Form einer Nährlösung ausgestaltet sein kann, umfasst somit mehrere Medien oder Bestandteile, die für das Wachstum des Organismus (u.U. der Organismen) und/oder seinen (u.U. jeweiligen) Stoffwechsel wichtig sind. Durch Anpassen der Volumenverhältnisse dieser Medien innerhalb des jeweiligen Reaktionsgefäßes kann der Roboter unterschiedliche Zusammensetzungen des Kulturmediums in dem jeweiligen Reaktionsgefäß herstellen; hierbei kann unter Umständen die Instruktion des Controllers so weit gehen, dass einzelne Bestandteile des Kulturmediums überhaupt nicht mehr in das jeweilige Reaktionsgefäß abgegeben werden. Mit anderen Worten kann sich die genaue Zusammensetzung des Kulturmediums in Bezug auf Inhaltsstoffe und ihre (Massen- /Volumen-)Verhältnisse im Laufe einer von der Apparatur durchgeführten Versuchssequenz (diese kann eine oder mehrere Versuchsreihen umfassen) verändern. Im Extremfall kann die Apparatur mit einer ersten Zusammensetzung die Versuchssequenz starten und an deren Ende eine Zusammensetzung des Kulturmediums als Optimum präsentieren, welches sich grundlegend von dem initial verwendeten Kulturmedium unterscheidet. Es sei noch erwähnt, dass sich die Anzahl N an gerade parallel ablaufenden Kultivierungsversuchen im Rahmen einer Versuchssequenz ändern, insbesondere abnehmen, kann.

Führt der Roboter die Instruktion aus, so stellt er in jedem der N Reaktionsgefäße eine jeweilige in der Instruktion festgelegte Zusammensetzung des Kulturmediums her, als Startbedingung für eine neue Versuchsreihe aus N Kultivierungsversuchen und/oder er stellt ein bestimmtes Verhältnis (bezogen auf die Biomassekonzentration) zwischen den wenigstens zwei Organismen im jeweiligen Reaktionsgefäß her. Die Instruktion umfasst also mindestens einen Datensatz aus N, insbesondere (aber nicht zwingend) unterschiedlichen, Zusammensetzungen und/oder Verhältnissen.

Diese erfindungsgemäße Apparatur lässt sich insbesondere einsetzen, um eine computer-implementierte Steuerung einer Vielzahl an (in den einzelnen Reaktionsgefäßen, insbesondere gleichzeitig, ablaufenden) biologischen Prozessen zu implementieren, um so die Kultivierung eines zu untersuchenden Organismus in dem Kulturmedium zu untersuchen und automatisiert zu optimieren. Denn mittels der mit Hilfe des Roboters vorgegebenen Zusammensetzung des Kulturmediums, in welchem der Organismus jeweils während der jeweiligen Versuchsreihe wächst oder kultiviert wird (also z.B. zumindest einen gewünschten Stoffwechselprozess kontinuierlich ausführt), können die Wachstums- und/oder Lebensbedingungen als Wirkparameter gezielt eingestellt und damit der biologische Prozess gesteuert werden. Ebenso kann ein Verhältnis der wenigstens zwei Organismen als ein Wirkparameter aufgefasst werden, da auch dieses entscheidend für die Wachstums- und/oder Lebensbedingungen der jeweiligen Organismen sein kann und/oder für ein zu erreichendes Prozessziel, beispielsweise einen bestimmten Stoffwechsel.

Das Kulturmedium, welches der Kultivierung des zu untersuchenden Organismus dient, kann also von der Apparatur gezielt eingestellt werden, um einen bestimmten gewünschten Wachstumsprozess des Organismus zu erzielen oder aber um zumindest eine bestimmte gewünschte Stoffwechselaktivität des Organismus (ggf. ohne größeres oder ohne jegliches Wachstum des Organismus) aufrechtzuerhalten. Die Erfindung ist somit nicht auf das computer-implementierte Steuern/Regeln von biologischen Wachstumsprozessen beschränkt, sondern kann ganz allgemein zur Steuerung eines biologischen Prozesses eingesetzt werden, der von dem Organismus (auf Basis des von der Apparatur eingestellten Kulturmediums) ausgeführt wird.

Dieser Prozess kann auch in einer Ko-Existenz von wenigstens zwei Organismen bestehen, bei der alle diese Organismen erfolgreich (z.B. in einer Symbiose) kultiviert werden.

Hierbei kann die Apparatur (genauer der Controller) auch noch weitere Wirkparameter, wie etwa die eine globale oder jeweilige Temperatur des einzelnen Reaktionsgefäßes und/oder eine globale oder jeweilige Bestrahlung des Reaktionsgefäßes mit Licht (z.B. im Fall eines Organismus der Photosynthese betreibt) und/oder eine globale oder aber jeweilige Zusammensetzung einer Gasatmosphäre in dem Reaktionsgefäß, auf Basis der jeweiligen computer-implementiert ermittelten Bewertung der gemessenen Prozessantwort anpassen, also für die nachfolgende Versuchsreihe in angepasster (optimierter) Form vorgeben. Unter "global" kann hier verstanden werden, dass der jeweilige Wirkparameter für alle N Reaktionsgefäße gemeinsam angepasst wird, etwa indem eine Gasatmosphäre, in welcher sich die Reaktionsgefäße befinden, für alle Gefäße gemeinsam angepasst wird.

Die Instruktionsanweisung kann somit wenigstens einen weiteren Wirkparameter (Temperatur des Reaktionsgefäßes oder anzuwendende Bestrahlungsstärke oder Zusammensetzung einer Gasatmosphäre) umfassen, der/die von der Apparatur während der laufenden Versuchsreihe für jedes der N Reaktionsgefäße (einzeln) vorgegeben werden soll (und wird). Die Instruktionsanweisung umfasst also mindestens N unterschiedliche Zusammensetzungen des Kulturmediums, die jeweils von dem Roboter für die neue Versuchsreiche in den N Reaktionsgefäßen angelegt werden sollen.

Es kann beispielsweise auch vorgesehen sein, dass einzelne der N Zusammensetzungen gleich sind, aber ein anderer Wirkparameter, beispielsweise die Temperatur desjenigen Reaktionsgefäßes, in dem diese Zusammensetzung zur Kultivierung des Organismus gerade verwendet wird, oder das besagte Verhältnis der wenigstens zwei Organismen variiert wird. Eine solche Variation eines weiteren Wirkparameters wie etwa der Temperatur oder des besagten Verhältnisses kann dann also zwischen wenigstens zwei Reaktionsgefäßen bestehen, die dieselbe Zusammensetzung des Kulturmediums aufweisen (zumindest während des gerade ablaufenden Kultivierungsversuchs).

Durch den erfindungsgemäßen Ansatz kann die Apparatur, genauer der Controller, somit selbständig erlernen, wie der Organismus / die Organismen in dem Kulturmedium optimal kultiviert werden kann. Die ermittelte optimierte Zusammensetzung des Kulturmediums und/oder die optimierten Wirkparameter (etwa ein optimiertes Verhältnis von zwei Organismen) kann dann, vorzugsweise in Form eines digitalen Datensatzes, von der Apparatur am Ende der Versuchssequenz bereit gestellt werden.

Hierbei kann der Controller auf Basis der Bewertungen insbesondere selbst entscheiden, wie viele Versuchsreihen (innerhalb der gesamten Versuchssequenz aus mehreren aufeinander folgenden Versuchsreihen) nacheinander durchgeführt werden. Somit kann der Controller den Abbruch der Versuchssequenz selbständig festlegen und damit die Zeitdauer, die für die gesamte Versuchssequenz benötigt wird.

Die Zielvorgabe gibt dabei eine gewünschte Prozessantwort vor, die bei Kultivierung des Organismus in dem jeweiligen Kulturmedium erzielt werden soll, beispielsweise ein bestimmter Zuwachs an Biomasse des Organismus (durch Vermehrung des Organismus selbst) oder eine bestimmte Wachstumsrate (Zuwachs an Biomasse pro Zeitintervall) oder eine bestimmte Stoffproduktion/Zeit oder einen bestimmten Stoffverbrauch/Zeit, die der Organismus liefern soll, oder einen bestimmten Zustand des Organismus, der besonders lange aufrechterhalten werden soll. Da die optimale Zusammensetzung des Kulturmediums aber zunächst unbekannt ist, kann die Apparatur die Zusammensetzungen des Kulturmediums für eine erste Reihe von N parallel in den N einzelnen Reaktionsgefäßen ablaufenden Kultivierungsversuchen (d.h. der Organismus wird im jeweiligen Kulturmedium innerhalb des jeweiligen Reaktionsgefäßes kultiviert) zunächst zufällig wählen, also initial (d.h. in der ersten Versuchsreihe der Sequenz) zufällige Startbedingungen festlegen. Gleiches gilt auch für das Verhältnis der wenigstens zwei Organismen.

Auf Basis der gemessenen Prozessantworten/Prozesszustände dieser ersten Reihe von Kultivierungsversuchen, kann die Apparatur, genauer der Controller, dann die Startbedingungen für eine nachfolgende Reihe an N Kultivierungsversuchen, die mit demselben Organismus in denselben N Reaktionsgefäßen automatisiert von der Apparatur durchgeführt wird, vorgeben. Hierzu kann der Controller entsprechende jeweilige N Instruktionsanweisungen (diese können in einer, insbesondere digitalen, Instruktion zusammengefasst sein) zum Herstellen von N angepassten Zusammensetzungen des Kulturmediums an den Roboter übermitteln, damit dieser diese N angepassten Zusammensetzungen des Kulturmediums in den einzelnen Reaktionsgefäßen herstellt.

Als geeignete Zielgröße, die als Zielvorgabe im Sinne der Erfindung verwendet werden kann, kann beispielsweise ein bestimmtes Niveau oder ein bestimmter Zuwachs an Biomasse vorgegeben sein.

Das Wachstum von Organismen verläuft oftmals anhand einer S-Kurve. Daher kann die Zielvorgabe, anhand derer der Controller / die KI die jeweilige Prozessantwort/den jeweiligen Prozesszustand bewertet, beispielsweise einem zu erreichenden Plateau dieser S-Kurve entsprechen oder beispielsweise auch einer maximale Steigung der S-Kurve; mit anderen Worten kann die Zielvorgabe beispielsweise eine bestimmte Biomassekonzentration (z.B. gemessen als eine Volumenkonzentration des Organismus innerhalb des Reaktionsgefäßes / des Kulturmediums) oder eine bestimmte zu erzielende Wachstumsrate (z.B. bestimmt aus den Messungen der Prozessantwort als Zuwachs an Biomasse in Gramm pro Zeit und Volumen) sein. Ferner kann die Zielvorgabe auch eine spezifische Wachstumsrate p (typischerweise für eine Monokultur), insbesondere ein zeitlicher Verlauf der Wachstumsrate µ, sein. Ein solcher Messparameter ist nämlich hervorragend geeignet, um mikrobiologische Prozesse zu steuern. Daher wird die Wachstumsrate µ auch häufig in der Bioverfahrenstechnik messtechnisch erfasst. Wird dabei beispielsweise ein Flowcytometer in der Prozessmessvorrichtung eingesetzt, kann es möglich sein, auch die jeweiligen einzelnen Wachstumsraten von zwei Organismen, die gleichzeitig in einem der Reaktionsgefäße kultiviert werden, als zwei Prozessantworten zu erfassen.

Als Messgröße, die mittels der Prozessmessvorrichtung gemessen wird, kann bevorzugt eine optische Dichte (beispielsweise um eine optische Trübungsmessung zu realisieren) und/oder ein Intensitätswert von (von dem Kulturmedium und dem Organismus) rückgestreutem Licht verwendet werden. Die optische Dichte oder Extinktion ist dabei ein Maß für die Abschwächung einer Strahlung nach Durchqueren eines Mediums. Hierbei kann das Kulturmedium vorzugsweise so beschaffen/gewählt sein, dass es selbst keine signifikante Trübung/optische Absorption oder Rückstreuung des bei der Messung eingesetzten Lichts zeigt. Beispielsweise kann erst nach Einbringen eines Organismus und dem nachfolgenden Wachstum dieses Organismus ein optisches Messsignal entstehen, etwa weil der Organismus das Kulturmedium zunehmend trübt und/oder eine optische Rückstreuung verursacht.

Hierbei besteht die Annahme, dass je höher die gemessene optische Trübung und/oder je höher die Intensität des zurückgestreuten Lichts ausfällt, umso höher ist die Biomassekonzentration in dem jeweiligen Reaktionsgefäß. Durch eine solche optische Erfassung der Änderung der Biomassekonzentration in dem jeweiligen Reaktionsgefäß (als einer möglichen Prozessantwort, die von dem Organismus selbst erzeugt wird), kann beispielswese ein bestimmtes gewünschtes Wachstum/Zeit, also eine Ziel-Wachstumsrate, als Zielvorgabe mit dieser gemessenen Prozessantwort verglichen werden, um letztere zu bewerten (die Bewertung könnte beispielsweise lauten: "zu hohe Wachstumsrate" oder "zu niedrige Wachstumsrate").

Anhand einer solchen jeweiligen Bewertung kann der Controller, insbesondere mittels einer künstlichen Intelligenz, wie noch genauer beschrieben wird, bewerten, ob die jeweilige Zusammensetzung günstig ist, um die Prozessantwort und/oder den Prozesszustand näher an die Zielvorgabe heranzuführen, oder eher ungünstig. Entsprechend können dann die günstigen Zusammensetzungen in einer nachfolgenden Versuchsreihe genauer untersucht werden, etwa in dem weitere, unter Umständen kleinere oder zusätzliche, Anpassungen des Kulturmediums in dem jeweiligen Reaktionsgefäß von der Apparatur vorgenommen werden. Hierbei kann sich die Anzahl N an parallel ablaufenden Kultivierungsversuchen selbstverständlich auch verringern, beispielsweise wenn nur noch eine Subauswahl näher untersucht wird, oder aber vergrößern, etwa wenn feinere Abstufungen der vorgenommenen Anpassungen im jeweiligen Kultivierungsversuch erforderlich werden.

Wie noch genauer erläutert werden wird, ist es vorteilhaft, wenn die Reaktionsgefäße zwischen den einzelnen Versuchsreihen (jede Versuchsreihe besteht aus N einzelnen parallel ablaufenden Kultivierungsversuchen), die eine Versuchssequenz bilden, von der Apparatur gereinigt und desinfiziert werden, sodass bei Herstellung der angepassten Zusammensetzungen erneut kontrollierte Startbedingungen für die nächste Versuchsreihe vorliegen.

Zudem kann jedes der Reaktionsgefäße zum Starten des jeweiligen Kultivierungsversuchs neu mit dem Organismus beimpft werden, indem eine definierte Menge eines Inokulums des Organismus, mit bekannter Volumenkonzentration des Organismus, in das jeweilige Reaktionsgefäß von dem Roboter eingebracht wird. Diese N-Beimpfungen der N-Reaktionsgefäße zu Beginn einer jeden neuen Versuchsreihe können also ebenfalls vollautomatisiert durchgeführt werden.

Die Reaktionsgefäße können beispielsweise durch die einzelnen Mikrotiter-Kavitäten (wells, insbesondere micro-wells) einer Mikrotiterplatte (microwell-plate) realisiert sein. Mit anderen Worten können also insbesondere räumlich voneinander getrennte, halboffene Reaktionsgefäße verwendet werden.

Hierbei können die einzelnen Reaktionsgefäße Volumina von wenigen mL aufweisen. Dadurch kann die Apparatur kompakt ausgestaltet werden und gleichzeitig ein hoher Durchsatz erzielt werden, weil zahlreiche Reaktionsgefäße (z.B. mindestens 6, mindestens 48 oder sogar mindestens 96 Reaktionsgefäße, insbesondere bei Verwendung mehrerer wellplates / mehrerer Bioreaktoren) gleichzeitig eingesetzt werden können. Mit der Apparatur lässt sich somit eine automatisierte Hochdurchsatzprüfung (HTS High-Throughput Screening) von unterschiedlichen Zusammensetzungen eines zu untersuchenden Kulturmediums realisieren, wobei dabei stets derselbe Organismus in den unterschiedlichen Zusammensetzungen kultiviert werden kann, oder wie erwähnt beispielsweise auch mehrere unterschiedliche Organismen. Jedes der Reaktionsgefäße kann somit nach Befüllung mit dem Nährmedium ein Reaktionsvolumen bereitstellen, in welchem der Organismus den gewünschten biologischen Prozess (Wachstum des Organismus selbst oder ein bestimmter Stoffwechselvorgang, der auf den Organismus zurückgeht) ausführen kann.

Die Reaktionsgefäße, in denen die Kultivierungsversuche stattfinden, können alternativ auch in einem Bioreaktor, insbesondere in einem Bioreaktorblock (beispielsweise bioREACTOR 48 von 2mag AG), ausgestaltet sein. Hierbei kann dann jedes Reaktionsgefäß durch einen geschlossenen Mini-Reaktor gebildet sein. Mit diesem Ansatz kann die Erfindung beispielsweise für die Optimierung einer Hochdurchsatz-Kultivierung (also beispielsweise eine mikrobielle Umwandlung organischer Stoffe durch probiotische Bakterien und/oder Pilze) im Bereich der Biotechnologie, Chemie und Pharmazie eingesetzt werden. Auch solche Bioreaktoren lassen sich mit Hilfe des Pipettierroboters der erfindungsgemäßen Apparatur vollautomatisiert betreiben. Und auch hier trägt die Miniaturisierung (Volumen der Reaktionsgefäßen von wenigen mL) zu einer signifikanten Material- und Kostenersparnis bei.

Bei solchen Bioreaktoren kann beispielsweise eine automatische Drehzahlüberwachung eines jeweiligen Rührstabs sowie eine nicht-invasive Echtzeitmessung von pH und Gelöstsauerstoff (dissolved oxygen (DO)) realisiert sein, ebenso wie eine Begasung und Durchmischung der einzelnen Reaktionsgefäße, beispielsweise durch gasinduzierende, induktiv angetriebene Magnetrührorgane. Es versteht sich, dass solche Mess- oder Steuergrößen mögliche Wirkparameter sind, die ebenfalls von der erfindungsgemäßen Apparatur, genauer dem Controller, computer-implementiert während einer Versuchsreihe erfasst und/oder vorgegeben werden können. Somit kann der Controller dazu eingerichtet sein, auf Basis der N Bewertungen computer-implementiert wenigstens einen Wirkparameter eines Bioreaktors vorzugeben (wobei der Bioreaktor die N Reaktionsgefäße bereitstellt) und/oder wenigstens einen Messparameter dieses Bioreaktors automatisiert zu erfassen. Selbstverständlich können in einer erfindungsgemäßen Apparatur auch mehrere solcher Bioreaktoren parallel betrieben und/oder zentral von dem Controller gesteuert werden.

Es versteht sich, dass die Apparatur mehrere Medien-Behälter aufweisen kann, in welchen Bestandteile, im Folgenden als Medien bezeichnet, des Kulturmediums vorhaltbar sind. Dadurch kann die Apparatur, genauer der besagte Roboter, aus diesen Medien jeweilige unterschiedliche Zusammensetzungen des Kulturmediums zusammenstellen, insbesondere innerhalb des jeweiligen Reaktionsgefäßes.

Schließlich kann die Apparatur bevorzugt auch ein Filter- oder sonstiges Modul aufweisen, mit dem eine Atmosphäre, in welcher sich die Reaktionsgefäße befinden, kontrolliert werden kann. Beispielsweise kann eine sterile Kopfraumbegasung oberhalb der Reaktionsgefäße / des Bioreaktors implementiert sein, etwa um Kreuz- und Fremdkontaminationen zu verhindern. So können beispielsweise aerobe wie anaerobe Mikroorganismen in der Apparatur kultiviert werden.

Erfindungsgemäß kann die Aufgabe auch durch weitere vorteilhafte Ausführungen gemäß den Unteransprüchen gelöst werden, die im Folgenden im Detail erläutert werden sollen:
Wie erwähnt, kann der Roboter bevorzugt als ein Pipettier-Roboter ausgestaltet sein, also beispielsweise mit einer elektronisch ansteuerbaren Pipette, mit der Flüssigkeiten (automatisiert / elektronisch geregelt) aufgenommen und dispensiert werden können. Insbesondere kann der Pipettierroboter einen beweglichen Pipettier-Arm aufweisen, der dazu eingerichtet ist, über dem jeweiligen der insgesamt N Reaktionsgefäße positioniert zu werden. Eine solche Ausgestaltung erlaubt es, einzelne Medien/Komponenten des Kulturmediums in das jeweilige Reaktionsgefäß zu dispensieren und/oder das jeweilige Kulturmedium aus dem jeweiligen Reaktionsgefäß mithilfe des Pipettier-Roboters abzusaugen. Alternativ hierzu kann der Roboter aber beispielweise auch ohne Pipette, etwa mit Hilfe eines Dispensier- und/oder Absaugschlauchs, ausgestaltet sein.

Bevorzugt ist dabei vorgesehen, dass die Apparatur ein Sterilisationsmittel aufweist zum automatisierten Sterilisieren einer Pipettenspitze oder eines Dispensierschlauchs des Pipettier-Roboters. Dieses Sterilisationsmittel kann beispielsweise mittels einer Reinigungsstation realisiert sein, in welcher der Pipettier-Roboter die Pipettenspitze von Zeit zu Zeit reinigen kann, sodass die Pipettenspitze automatisiert steril gehalten werden kann. Dadurch können beispielsweise Quer-Kontaminationen zwischen den einzelnen Reaktionsgefäßen, die die Versuchsreihe empfindlich stören würden, wirksam vermieden werden.

Es kann hierbei vorgesehen sein, dass der Roboter die einzelnen Bestandteile/Komponenten/Medien des Kulturmediums aus Vorratsbehältern der Apparatur entnehmen und in genau dosierter Menge in das jeweilige Reaktionsgefäß pipettieren/dispensieren kann, um so eine bestimmte Zusammensetzung des Kulturmediums in dem Reaktionsgefäß herzustellen.

Eine besonders bevorzugte Ausgestaltung sieht vor, dass der Controller den Roboter mithilfe einer künstlichen Intelligenz steuert. Dies kann insbesondere derart implementiert sein, dass die Apparatur anhand der gemessenen Prozessantworten und/oder anhand der gemessenen Prozesszustände im Laufe mehrerer zeitlich aufeinanderfolgender Versuchsreihen selbstständig erlernt, wie der Roboter eine optimierte Zusammensetzung des Kulturmediums herstellen kann.

Die künstliche Intelligenz (KI) kann dabei auch wenigstens eine nicht-technische Randbedingung berücksichtigen, beispielsweise den Preis einzelner Bestandteile des Kulturmediums. D.h. der Controller / die KI kann die vorzugebende Zusammensetzung des Kulturmediums und/oder die jeweiligen Anteile der unterschiedlichen Organismen anhand mehrerer Kriterien, inklusive dem sich ergebenden Preis des Kulturmediums, im Laufe der Versuchssequenz optimieren. Dies ist möglich, wenn die nicht-technischen Randbedingungen als Teil der Zielvorgabe (Minimiere den Preis! Bevorzuge Organismus X!) bei der Bewertung der Prozessantwort (und damit auch der ihr zugrundeliegenden Zusammensetzung des Kulturmediums) vom Controller berücksichtigt werden.

Was genau eine optimierte Zusammensetzung des Kulturmediums ist, kann also auf Basis der gemessenen Prozessantwort und gegebenenfalls auf Basis weiterer Randbedingungen vom Controller selbst entschieden werden: Je näher die Apparatur die Prozessantwort an die Zielvorgabe herangeführt hat (durch entsprechende Anpassung der Zusammensetzung und gegebenenfalls von weiteren Wirkparametern, also Wirkfaktoren, die die Kultivierung des Organismus in dieser Zusammensetzung des Kulturmediums zusätzlich beeinflussen), umso mehr hat sich die Zusammensetzung einem Optimum genähert. Wird keine signifikante Verbesserung der Prozessantwort mehr in nachfolgenden Versuchsreihen erzielt, kann der Controller selbständig entscheiden, die Versuchssequenz abzubrechen oder beispielsweise noch versuchen, ob ähnliche gute Ergebnisse mit einer anderen Zusammensetzung erzielbar sind, die in Bezug auf eine nicht-technische Randbedingung optimiert ist. Hierfür kann zum Beispiel ein bestimmtes Abbruchkriterium in Bezug auf ein Delta zwischen derjenigen der N gemessenen Prozessantworten, die der Zielvorgabe am nächsten kommt, und der Zielvorgabe selbst, vorab festgelegt sein.

Wie bereits erwähnt, kann der Controller über eine künstliche Intelligenz (KI) verfügen, die insbesondere auf Basis maschinellen Lernens beruhen kann oder einen Algorithmus maschinellen Lernens implementiert. Mithilfe dieser künstlichen Intelligenz kann der Controller aus den jeweils gemessenen Prozessantworten selbstständig, also insbesondere ohne menschliches Eingreifen, eine optimierte Zusammensetzung des Kulturmediums erlernen und diese optimierte Zusammensetzung ausgeben (z.B. in Form eines digitalen Datensatzes), z.B. am Ende einer mit der Apparatur durchgeführten Sequenz von mehreren (zeitlich aufeinander folgenden) Versuchsreihen.

Die Anpassung der jeweiligen Zusammensetzung des Nährmediums, welches für eines der Reaktionsgefäße (insbesondere im Rahmen eines einzelnen Kultivierungsversuchs, innerhalb einer Versuchssequenz, die von der Apparatur durchgeführt wird) vorgesehen ist, kann somit mittels einer künstlichen Intelligenz (KI) computerimplementiert erfolgen. Hierfür können insbesondere Methoden des maschinellen Lernens, zum Beispiel eine Methode des mehrschichtigen Lernens (deep learning) und/oder künstliche neuronale Netze (KNN) eingesetzt werden, d.h. von dem Controller implementiert sein.

Der Ansatz, den die Erfindung verfolgt, besteht somit unter anderem darin, dass die künstliche Intelligenz, beispielsweise mithilfe maschinellen Lernens, selbstständig erlernt, wie die optimale Zusammensetzung des Kulturmediums aussehen sollte, um die gewünschte Prozessantwort mit dem Organismus zu erzielen. Hierbei muss die KI aber die zugrundeliegenden kausalen Zusammenhänge, also die biochemischen Wechselwirkungen zwischen Kulturmedium und Organismus, gerade nicht verstehen; vielmehr lernt die KI aus empirischen Beobachtungen, nämlich den in den Versuchsreihen gemessenen Prozessantworten.

Wie bereits eingangs erwähnt, kann die Apparatur bevorzugt einen Inokulum-Bioreaktor umfassen. In diesem Bioreaktor kann nämlich ein Inokulum des zu kultivierenden Organismus bereitgehalten werden oder bereitgehalten sein. Hierbei kann der Roboter insbesondere dazu eingerichtet sein, eine Probe des Inokulums aus dem Inokulum-Bioreaktor zu entnehmen und das jeweilige Reaktionsgefäß mit dieser Probe zu beimpfen, was typischerweise am Anfang einer Versuchsreihe durchgeführt werden kann.

Unter einem Inokulum (oder auch Vorkultur) wird im Allgemeinen eine Impfkultur, also eine bestimmte Menge einer Reinkultur eines (Mikro-)Organismus verstanden, die zur Auf- und Weiterzucht verwendet werden kann. Im Rahmen der Erfindung kann unter Inokulum insbesondere eine Impfkultur verstanden werden, die neben dem zu kultivierenden biologischen Organismus auch ein (Inokulum-)Kulturmedium umfasst, in welchem der Organismus wächst beziehungsweise kultivierbar ist. Hierbei kann das Kulturmedium des Inokulums abweichend von demjenigen Kulturmedium gewählt sein, dessen Zusammensetzung mit der Apparatur gerade optimiert werden soll.

Die Erfindung hat insbesondere erkannt, dass in vielen biologischen Organismen zwar typischerweise Mutationen auftreten können, wenn diese über längere Zeiträume (insbesondere als ein Inokulum) kultiviert werden. Aufgrund der hohen Geschwindigkeit, die mit der erfindungsgemäßen Apparatur bei der Durchführung der Versuchssequenz erzielt werden kann, beträgt die Gesamtdauer, die benötigt wird, um mithilfe der KI eine optimierte Zusammensetzung des Kulturmediums zu bestimmen, jedoch nur einige Tage bis wenige Wochen, je nach Organismus. In solchen Zeitraumräumen treten aber erfahrungsgemäß noch keine relevanten Mutationen auf, sodass dies den Ansatz der Erfindung gerade nicht behindert.

Um eine Verunreinigung des Inokulums mit anderen Organismen zu vermeiden, kann die Entnahme der Probe insbesondere durch eine Sterilbarriere (z.B. in Form einer Membran) hindurch durchgeführt werden, die ein Inokulum-Reaktionsgefäß des Inokulum-Bioreaktors vor äußeren Einflüssen schützt. Auf dieselbe Weise können bevorzugt auch die Reaktionsgefäße, in welchen die Kultivierungsversuche durchgeführt werden, durch eine Sterilbarriere vor äußeren Einflüssen geschützt sein. Ferner kann wie erläutert auch die jeweilige Zusammensetzung einer Atmosphäre, in welcher sich das jeweilige Reaktionsgefäß befindet, etwa mit einem HEPA-Modul, kontrolliert werden/sein.

Beispielsweise kann der Roboter dazu eingerichtet sein, eine bestimmte Höchstmenge, beispielsweise höchstens 5 Vol.%, des im Bioreaktor bereitgehaltenen Inokulums als eine Probe zu entnehmen und diese Probe auf die N Reaktionsgefäße, in jeweils genau dosierter und gleicher Menge, zu verteilen. Während die Versuchsreihe läuft, beispielsweise über ein Zeitintervall von 12 h, kann der Bioreaktor die aus dem Inokulum entnommene Menge des Organismus wiederauffüllen, indem der Organismus in dem Inokulum weiter unter geeigneten Bedingungen kultiviert wird. Damit kann am Ende der Versuchsreihe erneut ausreichend Biomasse des Organismus in dem Inokulum innerhalb des Bioreaktors zur Verfügung stehen, um eine erneute Entnahme einer (z.B. gleich großen) Probe zu ermöglichen. Es versteht sich, dass dieser Prozess viele Male nacheinander wiederholt werden kann, über die gesamte Dauer einer Versuchssequenz aus mehreren Versuchsreihen.

Es sind hierbei auch Anwendungen denkbar, in welchen in dem Inokulum mehrere unterschiedliche Organismen vorhanden sind, die gemeinsam von der Apparatur in dem Kulturmedium kultiviert werden sollen. Beispielsweise kann dieser Ansatz genutzt werden, um optimale Kultivierungsbedingungen für symbiotische Paare aus Organismen zu untersuchen.

Die Entnahmemenge aus dem Inokulum kann wie erwähnt unterhalb einer bestimmten Höchstmenge von beispielsweise 5 % des Volumens des Bioreaktorsystems liegen. So kann beispielsweise innerhalb von 12 Stunden, in denen eine Versuchsreihe durchgeführt wird, der geregelte Bioreaktor die ursprünglich vorhandene Dichte des Organismus (beispielsweise eine Zelldichte oder optisch indirekt als eine optische Dichte erfasst) in dem Inokulum wiederherstellen, sodass erneut konstante Ausgangs-Bedingungen für die nächste Versuchsreihe bestehen.

Nimmt die Zelldichte in dem Inokulum beispielsweise überhand, so kann das Inokulum automatisiert, beispielsweise mithilfe des Roboters oder aber mittels einer Pumpe des Bioreaktors, verdünnt werden, wodurch die Dichte des Organismus wieder abgesenkt werden kann. Nimmt die Dichte (z.B.: im Bioreaktor sensorisch erfasste Zelldichte oder optische Dichte) hingegen ab, können, insbesondere über Pumpen des Bioreaktors oder mit Hilfe des Roboters, frische Medien zugeführt werden, um das Wachstum des Inokulums wieder anzukurbeln. Der Einsatz eines Pipettierroboters zum Einstellen der Biomassekonzentration des Organismus im Inokulum bietet sich insbesondere an, wenn mehrere unterschiedliche Inokula in der Apparatur bereit gehalten werden sollen.

Zudem kann auch die Temperatur dieses separaten Bioreaktors geregelt werden/sein und es kann vorgesehen sein, dass der Organismus in einer Nährlösung des Inokulums kontinuierlich (z.B. mittels eines Magnetrührers) durchmischt und agitiert wird. Diese Vorkehrungen führen dazu, dass in jeder Versuchsreihe der durchgeführten Versuchssequenz immer stets die gleichen Startbedingungen herrschen in Bezug auf die Qualität und Dichte/Biomassekonzentration des Organismus in dem Inokulum. Zudem ist hierfür die Reinheit des jeweiligen Reaktionsgefäßes wichtig, welches ja vor Start der aktuellen Versuchsreihe (automatisiert mit Hilfe des Roboters) aufwendig gereinigt und desinfiziert werden kann, wie noch genauer beschrieben wird.

Es wird somit insbesondere vorgeschlagen, dass die Apparatur, vorzugsweise der Inokulum-Bioreaktor selbst, Mess- und Steuermittel umfasst, mit denen eine (insbesondere jeweilige) Biomassekonzentration in dem (insbesondere jeweiligen) Inokulum-Bioreaktor konstant gehalten werden kann.

Gemäß einer bevorzugten Ausgestaltung kann der Inokulum-Bioreaktor umfassen: wenigstens ein, vorzugweise temperaturgeregeltes, Inokulum-Reaktionsgefäß, in welchem das Inokulum über mehrere Tage, vorzugsweise über wenigstens zwei Wochen, vorhaltbar ist; und/oder wenigstens ein Inokulum-Einstellmittel zum Anpassen einer Zusammensetzung des Inokulums, insbesondere eines Kulturmediums des Inokulums. Ein solches Inokulum-Einstellmittel kann insbesondere durch wenigstens eine Pumpe zum Befördern eines Bestandteils eines Kulturmediums des Inokulums in das Inokulum-Reaktionsgefäß hinein und/oder durch wenigstens eine Pumpe zum Befördern eines Teils des Inokulums aus dem Inokulum-Reaktionsgefäß heraus, realisiert sein. Der Inokulum-Bioreaktor kann ferner wenigstens eine, vorzugweise optische, Inokulum-Messvorrichtung umfassen, die zum Messen einer Biomasse- /Volumenkonzentration des zu kultivierenden Organismus innerhalb des Inokulums bzw. innerhalb des Inokulum-Reaktionsgefäßes, eingerichtet ist. Es versteht sich, dass es hierfür vorteilhaft ist, wenn das Inokulum-Reaktionsgefäß (genauso wie die sonstigen Reaktionsgefäße der Apparatur) für eine bestimmte Messwellenlänge transparent ausgestaltet ist, wobei diese Messwellenlänge von der jeweiligen Messvorrichtung ausgewertet wird.

Unter "Vorhalten" des Inokulums kann hier insbesondere verstanden werden, dass das Inokulum in einem geeigneten Kulturmedium innerhalb des Inokulum-Reaktionsgefäß kontinuierlich von dem Bioreaktor unter geeigneten und gleichbleibenden physiologischen Bedingungen kultiviert wird, und zwar insbesondere ohne jeglichen menschlichen Eingriff von außen, also voll-automatisiert. Hierzu kann der Inokulum-Bioreaktor dazu eingerichtet sein, insbesondere durch entsprechendes Ansteuern der beschriebenen Temperaturregelung, dem wenigstens einen Einstellmittel und Auswertung der Messvorrichtung, eine Volumenkonzentration des Organismus innerhalb des Inokulums (innerhalb eines Zielintervalls) konstant zu halten. Geeignete Bioreaktoren, die als Teil der Apparatur verwendet werden können, um das Inokulum bereit zu halten, sind bereits kommerziell verfügbar.

Der Inokulum-Bioreaktor kann darüber hinaus einen Regler umfassen, der dazu eingerichtet ist, dass wenigstens eine Inokulum-Einstellmittel in Abhängigkeit einer mit der wenigstens einen Inokulum-Messvorrichtung durchgeführten Messung so anzusteuern, dass eine Volumenkonzentration des zu kultivierenden Organismus innerhalb des Inokulums innerhalb eines vorgegebenen Intervalls gehalten werden kann. Das Intervall gibt somit vor, in welchem Bereich die Volumenkonzentration des Organismus schwanken kann.

Ganz besonders vorteilhaft ist es ferner, wenn der Roboter dazu eingerichtet ist, die Reaktionsgefäße zu reinigen. Dies kann insbesondere nach Durchführung einer Versuchsreihe durchgeführt werden, wobei der Organismus in unterschiedlichen Zusammensetzungen des Kulturmediums in den einzelnen Reaktionsgefäßen während der Versuchsreihe kultiviert wurde. Das Reinigen kann vorzugweise durch Ausspülen und oder durch Desinfizieren geschehen, wobei letzteres insbesondere durch Spülen mit einem Desinfektionsmittel geschehen kann. Besonders bevorzugt ist es hierbei, wenn der Roboter beim Reinigen und Desinfizieren der Reaktionsgefäße einem festgelegten Ablaufplan folgt, also beispielsweise einem bestimmten Pipettierprotokoll, das/der mehrere Spülschritte und ein Warteintervall umfasst, in welchem das Desinfektionsmittel (beispielsweise eine Alkohollösung) auf das jeweilige Reaktionsgefäß einwirken kann.

Es kann beispielsweise vorgesehen sein, dass die Reaktionsgefäße in einem festgelegten zeitlichen Rhythmus von zum Beispiel 12 Stunden, insbesondere jeweils nach Ausführen einer Wachstumsphase des Organismus während einer 12h-Versuchsreihe und einer abschließenden optischen Vermessung des jeweiligen Reaktionsgefäßes zur Ermittlung der jeweiligen Prozessantwort, mit dem Roboter gespült, gereinigt und desinfiziert werden.

Beispielsweise kann nach einer erfolgten optischen Vermessung der N Reaktionsgefäße und anschließender computer-implementierten Bewertung einer Versuchsreihe, jedes einzelne der Reaktionsgefäße (z.B. 48 micro-wells einer Mikrotiterplatte) mit dem Pipettierroboter mehrfach mit sterilem Wasser gespült (Einspülen von Wasser, Ab-/Aussaugen; dann von vorne) und anschließend mit dem Pipettierroboter mit einer Alkohollösung desinfiziert werden, die eine gewisse Zeit einwirkt, bevor erneut gespült wird. Hierbei kann auf im Stand-der-Technik vorbekannte Pipettierprotokolle zurückgegriffen werden. Dadurch können die Reaktionsgefäße, die insbesondere durch eine gemeinsame Versuchsplattform wie etwa eine well-plate realisiert sein können, mehrmals verwendet werden. Dadurch muss, zumindest während einer Versuchssequenz, die Versuchsplattform nicht mehr ausgetauscht werden, selbst dann nicht, wenn diese eigentlich als Einweg-Artikel ausgestaltet ist. Dieses Vorgehen bannt zudem die Gefahr, dass Rückstände aus einer vorherigen Versuchsreihe in dem jeweiligen Reaktionsgefäß übrigbleiben, wodurch das Versuchsergebnis der nachfolgenden Versuchsreihe nicht mehr unmittelbar auf die darin verwendete neue Zusammensetzung des Kulturmediums zurückzuführen wäre und das Erlernen der optimalen Zusammensetzung/der optimalen Anteile der unterschiedlichen Organismen durch die KI erschwert oder gar unmöglich wäre.

Die besagte wenigstens eine Prozessmessvorrichtung kann bevorzugt (insbesondere jeweils) als eine optische Prozessmessvorrichtung ausgestaltet sein. Ferner ist es bevorzugt, wenn diese eine optische Transmissionsmessung und/oder eine optische Rückstreumessung realisiert.

Die Erfindung schlägt dabei insbesondere vor, dass die Prozessmessvorrichtung dazu eingerichtet ist, eine jeweilige Veränderung der Biomasse des Organismus (als der erzeugten Prozessantwort) in dem jeweiligen Reaktionsgefäß zu messen.

Alternativ oder aber ergänzend zur Erfassung der Veränderung der Biomasse als einer möglichen Prozessantwort, die der Organismus erzeugt, kann die Prozessmessvorrichtung aber auch dazu eingerichtet sein, durch den Organismus erzeugte Veränderungen von wenigstens einem Messparameter des Kulturmediums (beispielsweise pH-Wert und/oder Gelöstsauerstoff (DO)) in dem jeweiligen Reaktionsgefäß zu messen. Dieser Ansatz kann sich beispielsweise anbieten, wenn die Reaktionsgefäße wie beschrieben in einem Bioreaktor ausgestaltet sind und/oder wenn beispielsweise eine direkte Erfassung der Biomassekonzentration nicht möglich ist.

Gerade eine optische Rückstreumessung, bei der beispielsweise die Intensität von Licht gemessen wird, welches von dem Kulturmedium und dem darin befindlichen Organismus zurückgestreut wird (back scattered light), eignet sich hervorragend, um über eine weiten Messbereich ein näherungsweise lineares Messsignal zu erhalten, welches einen Rückschluss auf die momentane Volumenkonzentration des Organismus innerhalb des Reaktionsgefäßes / des Kulturmediums erlaubt. Hieraus kann der Controller das Wachstum des Organismus, insbesondere den zeitlichen Verlauf eines (z.B. über die Zeitspanne einer Versuchsreihe beobachteten) Zuwachses an Biomasse in dem Reaktionsgefäß, als Prozessantwort bestimmen.

Je nach Ausgestaltung der Apparatur kann diese beispielsweise nur über eine einzige Prozessmessvorrichtung verfügen, mit der jedes der Reaktionsgefäße, insbesondere sequenziell nacheinander, vermessen werden kann. Beispielsweise dann, wenn die Reaktionsgefäße als Kavitäten einer Mikrotiterplatte ausgestaltet sind, kann letztere, z.B. mithilfe eines Schrittmotors, so über die Prozessmessvorrichtung hinweg bewegt werden, dass die Prozessmessvorrichtung jede einzelne der Kavitäten optisch vermessen kann, ohne dass dabei die Prozessmessvorrichtung selbst bewegt werden muss. Alternativ ist es aber natürlich genauso möglich, die Reaktionsgefäße nicht zu bewegen und stattdessen die Prozessmessvorrichtung relativ zu den Reaktionsgefäßen an eine jeweilige Messstelle zu bewegen.

Alternativ hierzu kann auch jedes der Reaktionsgefäße über eine eigene Prozessmessvorrichtung verfügen, mit der dann eine jeweilige Prozessantwort, die der Organismus in dem jeweiligen Reaktionsgefäß erzeugt, insbesondere gleichzeitig und damit schneller, gemessen werden kann.

Die Erfassung der jeweiligen Prozessantwort kann dabei einmalig während einer durchgeführten Versuchsreihe, zum Beispiel an deren Ende, erfolgen, oder aber quasikontinuierlich, also beispielsweise in bestimmten zeitlichen Abständen während der Dauer der jeweiligen Versuchsreihe. Es kann somit unter Umständen mit der wenigstens einen Prozessmessvorrichtung insbesondere ein zeitlicher Verlauf der jeweiligen Prozessantwort, die von dem Organismus in dem jeweiligen Reaktionsgefäß erzeugt wird, erfasst / aufgezeichnet werden. Auch ein solcher zeitlicher Verlauf der Prozessantwort kann nämlich von dem Controller gegenüber einer komplexen Zielvorgabe, die beispielsweise bestimmte zeitliche Zielmarken umfassen kann, bewertet werden.

Ebenso kann, wie unten noch genauer erläutert wird, die Zusammensetzung des Kulturmediums während einer durchgeführten Versuchsreihe verändert werden, etwa durch quasikontinuierliche Zugabe wenigstens einer Komponente des Kulturmediums, und/oder das Verhältnis der beiden Organismen schrittweise während einer durchgeführten Versuchsreihe verändert werden.

Zur Lösung der genannten Aufgabe sind erfindungsgemäß bei einem Verfahren der eingangs genannten Art die Merkmale des unabhängigen Verfahrensanspruchs vorgesehen. Insbesondere wird somit erfindungsgemäß zur Lösung der Aufgabe bei einem Verfahren der eingangs beschriebenen Art vorgeschlagen, dass eine Anzahl von N räumlich getrennten Reaktionsgefäßen jeweils mit einer festgelegten Zusammensetzung des (zu optimierenden) Kulturmediums befüllt werden, was vorzugsweise mithilfe eines Roboters automatisiert erfolgen kann; dass ferner die N Reaktionsgefäße, vorzugsweise mit dem Roboter (der wie zuvor beschrieben ausgestaltet sein kann), mit einem Inokulum, welches den jeweiligen Organismus (also insbesondere einen der wenigstens zwei Organismen) umfasst, beimpft werden (Es versteht sich, dass bei gemeinsamer Kultivierung von wenigstens zwei Organismen wenigstens zwei solcher Einzel-Beimpfungen des jeweiligen Reaktionsgefäßes vorgenommen werden können); dass nachfolgend eine Versuchsreihe aus N Kultivierungsversuchen, die parallel, also vorzugweise gleichzeitig, in den N Reaktionsgefäßen mit dem Organismus/ den wenigstens zwei Organismen ablaufen, durchgeführt wird, wobei der jeweilige Organismus, insbesondere die wenigstens zwei Organismen gemeinsam, in jedem der N Reaktionsgefäße einen jeweiligen biologischen Prozess ausführt/ gemeinsam ausführen. Ferner ist vorgesehen, dass eine jeweilige Prozessantwort und/oder ein jeweiliger Prozesszustand, die/den der Organismus/die wenigstens zwei Organismen in dem jeweiligen Reaktionsgefäß erzeugt/erzeugen, mit wenigstens einer Prozessmessvorrichtung gemessen wird; dies kann insbesondere während und/oder am Ende der Versuchsreihe geschehen, wie bereits zuvor mit Bezug auf die Apparatur erläutert.

Schließlich ist vorgesehen, dass die N gemessenen Prozessantworten und/oder N gemessenen Prozesszustände Computer-implementiert, nämlich vorzugweise unter Einsatz einer künstlichen Intelligenz, jeweils anhand einer Zielvorgabe bewertet werden (wobei die Zielvorgabe bevorzugt für alle der biologischen Prozesse gilt) und dass auf Basis dieser N Bewertungen Computer-implementiert eine jeweilige Zusammensetzung des Kulturmediums, die in dem jeweiligen Reaktionsgefäß in einer nachfolgenden Versuchsreihe angelegt und/oder während eines nachfolgenden jeweiligen Kultivierungsversuchs verändert werden soll, mittels einer Instruktion computer-implementiert vorgegeben wird.

Soll hingegen das besagte Verhältnis zwischen den wenigstens zwei Organismen optimiert werden, so kann alternativ oder aber ergänzend zu dem zuletzt genannten Merkmal bei dem Verfahren vorgesehen sein, dass auf Basis dieser N Bewertungen Computer-implementiert das Verhältnis der wenigstens zwei unterschiedlichen Organismen, das in dem jeweiligen Reaktionsgefäß in einer nachfolgenden Versuchsreihe angelegt und/oder während eines nachfolgenden jeweiligen Kultivierungsversuchs verändert werden soll, mittels einer Instruktion computer-implementiert vorgegeben wird.

Bei diesem Verfahren kann bevorzugt eine erfindungsgemäß ausgestaltete Apparatur wie zuvor beschrieben oder gemäß einem der auf eine solche Apparatur gerichteten Ansprüche eingesetzt werden, um die zuvor erläuterten einzelnen Verfahrensschritte, insbesondere jeweils ohne menschliches Zutun und damit automatisiert, auszuführen. Das Verfahren kann also autonom von der Apparatur ausgeführt werden, wobei lediglich die notwendigen Medien und das Inokulum / die Inokula bereit gestellt sein müssen. Umgekehrt kann eine erfindungsgemäße Apparatur selbstverständlich zur Ausführung eines Verfahrens, wie es hierin beschrieben und/oder beansprucht ist, eingerichtet sein, insbesondere derart, dass die Apparatur das Verfahren autonom ausführt.

Beispielsweise kann bei dem Verfahren vorgesehen sein, dass der Roboter zu Beginn der nachfolgenden Versuchsreihe die Instruktion ausführt, indem er in jedem der N Reaktionsgefäße die jeweils durch die Instruktion vorgegebene Zusammensetzung des Kulturmediums herstellt und/oder das jeweils durch die Instruktion vorgegebene Verhältnis der wenigstens zwei unterschiedlichen Organismen herstellt (etwa durch Pipettieren von geeigneten Volumina von wenigstens zwei unterschiedlichen Inokula in das jeweilige Reaktionsgefäß).

Die zuvor beschriebenen Versuchsreihen können bei dem Verfahren iterativ als eine Sequenz aus mehreren zeitlich aufeinanderfolgenden Versuchsreihen durchgeführt werden.

Hierbei ist es bevorzugt, wenn schrittweise die Zusammensetzung des Kulturmediums auf Basis der jeweils in den einzelnen Versuchsreihen bestimmten N Bewertungen an ein Optimum herangeführt wird. Hierbei kann gleichzeitig oder aber getrennt hiervon auch das beschriebene Verhältnis der wenigstens zwei unterschiedlichen Organismen schrittweise optimiert werden.

So kann beispielsweise mithilfe des Verfahrens Schritt für Schritt eine optimierte Zusammensetzung des Kulturmediums ermittelt werden. Im Ergebnis kann so wenigstens eine der N gemessenen Prozessantworten näher an die Zielvorgabe heranrücken. In diesem Fall kann die Zusammensetzung des Kulturmediums, mit dem diese Prozessantwort erzeugt wurde, als die optimierte Zusammensetzung aufgefasst werden. Es versteht sich, dass dieses Konzept in analoger Weise genutzt werden kann, um das besagte Verhältnis der wenigstens zwei unterschiedlichen Organismen zu optimieren (entweder zusätzlich oder alternativ zur Optimierung der Zusammensetzung des Kulturmediums).

Beispielsweise kann auch vorgesehen sein, dass während eines laufenden Kultivierungsversuchs die jeweilige Zusammensetzung des Kulturmediums (mit dem das jeweilige Reaktionsgefäß befüllt wird) und/oder das jeweilige Verhältnis der wenigstens zwei (unterschiedlichen) Organismen (die innerhalb eines der Reaktionsgefäße gerade kultiviert werden) in dem jeweiligen Reaktionsgefäß verändert wird/werden. Eine solche Änderung kann also insbesondere während des laufenden Kultivierungsversuch vorgenommen werden. Die Veränderung des besagten Verhältnisses während des Versuchs kann auch zusätzlich zu einer Änderung der Zusammensetzung des Kulturmediums und/oder des besagten Verhältnisses jeweils zu Beginn einer neuen Versuchsreihe, d.h. jeweils zu Beginn eines jeweiligen Kultivierungsversuchs, vorgenommen werden. Dies kann beispielsweise dadurch geschehen, dass (insbesondere mit Hilfe des erwähnten Roboters) einzelne Komponenten des Kulturmediums und/oder wenigstens ein Inokulum von einem der wenigstens zwei erwähnten Organismen dem jeweiligen Reaktionsgefäß während eines gerade laufenden Kultivierungsversuchs hinzugefügt wird. Hierbei kann auch zuvor eine gewisse Menge des Kulturmediums samt dem/den darin enthaltenen Organismus/Organismen entfernt worden sein, insbesondere um so ein Überlaufen des Reaktionsgefäßes zu vermeiden und/oder um so das Verhältnis einfacher anpassen zu können. Mit solchen Ausgestaltungen können somit insbesondere Fed-Batch-Versuche automatisiert durchgeführt werden.

Die besagte Instruktion kann also insbesondere festlegen, wie die Zusammensetzung und/oder das besagte Verhältnis der wenigstens zwei Organismen während eines jeweiligen gerade ablaufenden Kultivierungsversuchs im jeweiligen Reaktionsgefäß verändert werden soll. Dieser Ansatz ist insbesondere für die Optimierung von Fed-Batch-Prozessen geeignet; hierunter versteht man Chargenprozesse, die als "Stapel", also zeitlich nacheinander, abgearbeitet werden, etwa durch ein schrittweises oder kontinuierliches Hinzuführen von Edukten. Mit anderen Worten kann also unter Umständen die Prozessführung bei einem erfindungsgemäßen Verfahren so ausgestaltet sein, dass die Zusammensetzung des Kulturmediums schrittweise oder sogar kontinuierlich während eines Kultivierungsversuchs verändert wird.

Bei dem Verfahren kann ferner vorgesehen sein, dass die Reaktionsgefäße, am Ende der jeweiligen Versuchsreihe, vorzugsweise mithilfe des Roboters, gereinigt und/oder desinfiziert werden. Dies kann insbesondere durch Ausspülen des Kulturmediums und nachfolgender Behandlung des jeweiligen Reaktionsgefäßes mit einem Desinfektionsmittel geschehen.

Schließlich kann bei dem Verfahren auch vorgesehen sein, dass das Inokulum, mit welchem die N Reaktionsgefäße zu Beginn einer jeden Versuchsreihe neu beschimpft werden, aus einem separaten (also separat von den Reaktionsgefäßen angeordnet) Inokulum-Bioreaktor entnommen werden. Diese Entnahme kann vorzugsweise mithilfe des Roboters geschehen, der zuvor beschrieben wurde. Ferner kann der Inokulum-Bioreaktor selbstverständlich wie zuvor beschrieben ausgestaltet sein. Es ist auch bevorzugt, dass dabei die Brutbedingungen des Inokulums und/oder eine Volumenkonzentration des Organismus in dem Inokulum automatisiert durch den Inokulum-Bioreaktor während einer gesamten Dauer einer Sequenz an durchgeführten Versuchsreihen konstant gehalten wird.

Die Erfindung wird nun anhand von Ausführungsbeispielen näher beschrieben, ist aber nicht auf diese beschränkt. Weitere Ausbildungen der Erfindung können aus der nachfolgenden Beschreibung eines bevorzugten Ausführungsbeispiels in Verbindung mit der vorherigen allgemeinen Beschreibung, den Ansprüchen sowie den zugehörigen Zeichnungen in naheliegender Weise gewonnen werden.

Bei der folgenden Beschreibung verschiedener Ausführungsformen der Erfindung erhalten Elemente, die in ihrer Funktion übereinstimmen, auch bei abweichender Gestaltung oder Formgebung übereinstimmende Bezugszahlen.

Es zeigt:
- Figur 1: eine schematische Ansicht einer ersten erfindungsgemäßen Apparatur, die dazu vorgesehen und eingerichtet ist, eine optimierte Zusammensetzung eines Kulturmediums zu ermitteln, in welchem ein biologischer Organismus kultiviert werden soll;
- Figur 2: eine schematische Ansicht einer zweiten möglichen Ausgestaltung einer erfindungsgemäßen Apparatur, mit der ein Verhältnis von zwei Mikroorganismen, die gemeinsam in einem Kulturmedium kultiviert werden sollen, autonom optimiert werden kann.

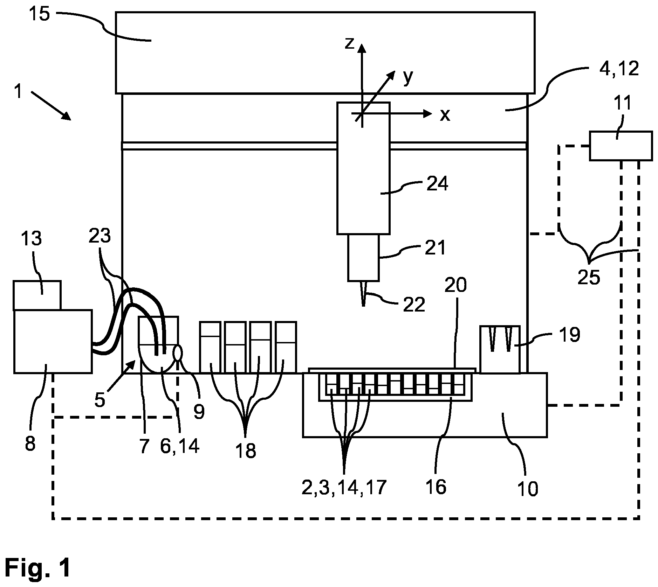

Die Figur 1 zeigt eine im Ganzen mit 1 bezeichnete erfindungsgemäße Apparatur die eine Mikrotiterplatte 16 aufweist mit zahlreichen in einem array angeordneten Mikrotiter-Kavitäten 17, die jeweils als ein Reaktionsgefäß 2 dienen. Mithilfe eines Pipettier-Roboters 12 der Apparatur 1, der über einen Controller 11 gesteuert wird, kann eine Pipette 21 in den drei Raumrichtungen x, y, z verfahren werden, sodass mithilfe des Pipettier-Roboters 12 unterschiedliche Zusammensetzungen eines Kulturmediums 3 in den jeweiligen Reaktionsgefäßen 2 herstellbar sind. Hierzu sind mehrere Vorratsgefäße 18 vorgesehen, die jeweils dem Aufbewahren einer bestimmten Medienkomponente dienen, wobei einzelne Mengen des jeweiligen Mediums mit der Pipette 21 automatisiert entnommen werden können. Der Pipettier-Roboter 12 kann so anhand einer Instruktion, die er von dem Controller 11 erhält, unterschiedliche Volumina des jeweiligen Mediums aus dem jeweiligen Vorratsgefäß 18 entnehmen und in das jeweilige Reaktionsgefäß 2 dispensieren, um so unterschiedliche Zusammensetzungen des Kulturmediums 3 in den einzelnen Reaktionsgefäßen 2 herzustellen.

Die Apparatur 1 umfasst ferner einen separaten Inokulum-Bioreaktor 5, in welchem ein Inokulum 6 eines zu kultivierenden Organismus 14 bei konstanten Bedingungen bereitgehalten wird. Hierzu umfasst der Inokulum-Bioreaktor 5 eine Messvorrichtung 9, mit der fortlaufend eine Volumenkonzentration des Organismus 14 innerhalb eines Inokulum-Reaktionsgefäßes 7 des Bioreaktors 5 messtechnisch erfasst werden kann. In Abhängigkeit dieser Messungen steuert ein Regler 13 ein Einstellmittel 8 an, mit dem die Zusammensetzung des Inokulums 6, genauer des Kulturmediums des Inokulums 6, in welchem der Organismus 14 fortwährend kultiviert wird, angepasst werden kann. Bei dem in Figur 1 gezeigten Beispiel ist das Einstellmittel 8 durch mehrere Pumpen realisiert, mit denen Bestandteile des Kulturmediums des Inokulums 6 über die illustrierten Schläuche 23 in das Inokulums-Reaktionsgefäß 7 nachgeliefert werden können. Der Controller 11 kann dabei auch steuernd und regelnd über die Steuerleitungen 25 auf den Regler 13 des Inokulum-Bioreaktors 5 einwirken, etwa um die Biomassekonzentration des Inokulums 6 zu verändern.

Darüber hinaus umfasst die Apparatur 1 ein Filter-Modul 15 in Form eines HEPA-Moduls, mit dem eine sterile Gas-Atmosphäre oberhalb der Reaktionsgefäße 2 bereitgestellt werden kann, was der Vermeidung von Kontaminationen der einzelnen Reaktionsgefäße 2 dient.

Mit der Apparatur 1 lässt sich für einen bestimmten Organismus 14, der in dem Inokulum 6 bei konstanter Biomassekonzentration bereitgehalten wird, die Zusammensetzung des Kulturmediums 3 optimieren, in welchem der Organismus 14 in den N Reaktionsgefäßen 2 der Mikrotiterplatte 16 kultiviert werden soll. Es versteht sich, dass hierbei das Kulturmedium 3 von demjenigen des Inokulums 6 abweichen kann.

Um die Zusammensetzung zu optimieren, führt die Apparatur 1 autonom wenigstens eine Versuchsreihe durch, die aus N Kultivierungsversuchen besteht. Hierzu stellt der Pipettier-Roboter 4,12 in jedem der N Reaktionsgefäße 2 eine jeweilige Zusammensetzung des Kulturmediums 3 (wie zuvor erläutert) her, wobei die initiale Zusammensetzung zunächst zufällig gewählt sein kann, aber automatisch von dem Controller 11 vorgegeben wird. Anschließend beimpft der Roboter 4 jedes der N Reaktionsgefäße 2 mit einer gewissen Menge des Inokulums 6, welches der Roboter 4 aus dem Inokulum-Bioreaktor 5 entnimmt. Um dabei Querkontaminationen zu vermeiden, fährt der Roboter 4 dabei jeweils zwischen den einzelnen Impfungen eine Reinigungsstation 19 an und reinigt dort seine Pipettenspitze 22. Die so steril gehaltene Pipettenspitze 22 kann so stets eine gleichbleibende Qualität des Inokulums 6 in das jeweilige Reaktionsgefäß 2 abgeben.

Nachdem alle Reaktionsgefäße 2 beimpft worden sind, führt die Apparatur 1 unter geeigneten Bedingungen über einen vorgegebenen Zeitraum hinweg eine Kultivierung des Organismus 14 durch. Beispielsweise können die Bedingungen (Temperatur, Druck, Atmosphäre) so gewählt sein, dass ein gewünschtes Wachstum des Organismus 14 und damit eine Zunahme an Biomasse in dem jeweiligen Reaktionsgefäß 2 zu erwarten ist. Am Ende dieser Wachstumsphase misst die Apparatur 1 autonom den Zuwachs an Biomasse des Organismus 14 in dem jeweiligen Reaktionsgefäß 2 als eine Prozessantwort mithilfe einer Prozessmessvorrichtung 10. Diese Prozessmessvorrichtung 10 ist bevorzugt optisch ausgestaltet und kann beispielsweise den Zuwachs an Biomasse im jeweiligen Reaktionsgefäß 2 mittels einer optischen Rückstreumessung oder einer Messung der jeweiligen optischen Dichte des in dem jeweiligen Reaktionsgefäß 2 befindlichen Kulturmediums erfassen. Besonders einfach gelingt dies beispielsweise dann, wenn die optische Prozessmessvorrichtung 10 einen statischen Messkopf aufweist und der Controller 11, über entsprechende Steuersignale und Aktorik, die Mikrotiterplatte 16 relativ zu dem statischen Messkopf verfährt, sodass die optische Prozessmessvorrichtung 10 jedes der N Reaktionsgefäße 2 jeweils einzeln (und sequentiell nacheinander) optisch vermessen kann. Alternativ könnten aber auch beispielsweise mehrere Prozessmessvorrichtungen 10 verwendet werden, um jeweils für jedes der N Reaktionsgefäße 2 eine eigene Messung durchzuführen (in diesem Fall können dann mehrere Messungen zeitgleich ausgeführt werden).

Der Controller 11 verarbeitet nun die N Messwerte, die im Rahmen der zuvor beschriebenen ersten Versuchsreihe für die N Reaktionsgefäße 2 mithilfe der Prozessmessvorrichtung 10 (als Prozessantworten oder Prozesszustände) gemessen wurden, und bewertet diese anhand einer Zielvorgabe, beispielsweise einer bestimmten gewünschten Zunahme der Biomassekonzentration pro Zeit, also einer bestimmten Wachstumsrate. Hierbei kann es beispielsweise vorkommen, dass bestimmte Zusammensetzungen des Kulturmediums 3, die in einigen der N Reaktionsgefäße 2 von dem Roboter 4 hergestellt worden sind, zu einer unerwünschten niedrigen und andere zu einer unerwünschten hohen Wachstumsrate führen. Der Controller verarbeitet nun diese N Bewertungen und gibt dann dem Roboter 4 in Form einer Instruktion vor, welche neuen N Zusammensetzungen des Kulturmediums 3 der Roboter 4 in dem jeweiligen der N Reaktionsgefäße 2 in einer nachfolgenden Versuchsreihe herstellen soll.

Um dabei wieder gleiche Ausgangsbedingungen für die nachfolgende neue Versuchsreihe aus N Kultivierungsversuchen zu schaffen, reinigt der Pipettier-Roboter 12 zunächst alle der N Reaktionsgefäße 2, indem er diese ausspült und anschließend mithilfe eines Desinfektionsmittels behandelt. Die so gereinigten und sterilisierten Reaktionsgefäße 2 stehen anschließend für eine erneute Befüllung mit dem Kulturmedium 3 zur Verfügung.

In der dann folgenden zweiten Versuchsreihe befüllt der Roboter 4,12 somit erneut alle N Reaktionsgefäße 2 mit je einer jeweiligen Zusammensetzung des Kulturmediums 3, die durch die beschriebene Instruktion des Controllers 11 vorgegeben ist. Anschließend beimpft der Roboter 4 alle N Reaktionsgefäße 2 erneut mit einer jeweiligen Probe des Inokulums 6, sodass nachfolgend eine neue Wachstumsphase desselben Organismus 14 in dem jeweiligen Reaktionsgefäß 2 ablaufen kann. Am Ende dieser Wachstumsphase, deren Zeitdauer auch von dem Controller 11 variiert werden kann, misst die Apparatur 1 erneut autonom mithilfe der Prozessmessvorrichtung 10 den jeweiligen Zuwachs an Biomassekonzentration in dem jeweiligen Reaktionsgefäß 2 und der Controller 11 nimmt erneut N Bewertungen dieser am Ende der zweiten Versuchsreihe gemessenen Prozessantworten vor. Anschließend gibt der Controller 11 erneut eine Instruktion vor, wodurch erneut eine Anpassung der jeweiligen Zusammensetzung des Kulturmediums 3 in dem jeweiligen Reaktionsgefäß 2 vorgegeben werden kann, dann für die dritte nachfolgende Versuchsreihe.

Auf diese Weise, also durch mehrmaliges sequentielles Ausführen solcher Versuchsreihen aus jeweils N parallel und gleichzeitig ablaufenden Kultivierungsversuchen, kann der Controller 11 Stück für Stück anhand der jeweiligen Bewertungen erlernen, wie die Zusammensetzung des Kulturmediums 3 optimal auf den Organismus 14 angepasst werden kann, sodass die gewünschte Zielvorgabe für die Wachstumsrate erzielt werden kann. Der Controller 11 verfügt hierzu über eine künstliche Intelligenz, die als ein neuronales Netz realisiert ist und ein Verfahren des maschinellen Lernens implementiert. Damit kann der Controller 11 aus den jeweils gemessenen Prozessantworten oder Prozesszuständen (beide Ansätze sind möglich) selbstständig - also ohne menschliches Eingreifen - die optimierte Zusammensetzung des Kulturmediums 3 erlernen kann. Am Ende einer durchgeführten Sequenz aus mehreren Versuchsreihen (die jeweils N Kultivierungsversuche umfassen) kann der Controller dann die gefundene optimierte Zusammensetzung des Kulturmediums 3 (vorzugsweise in digitaler Form) ausgeben. Es sei noch erwähnt, dass der Controller auf Basis der gemessenen Prozessantworten und/oder -zustände auch beispielsweise eine Vorgabe machen kann, wie die jeweilige Zusammensetzung des jeweiligen Kulturmediums während einer gerade ablaufenden Versuchsreihe aus N Versuchen jeweils verändert werden soll, etwa wenn im jeweiligen Reaktionsgefäß von der Apparatur 1 ein Fed-Batch-Prozess ausgeführt wird.

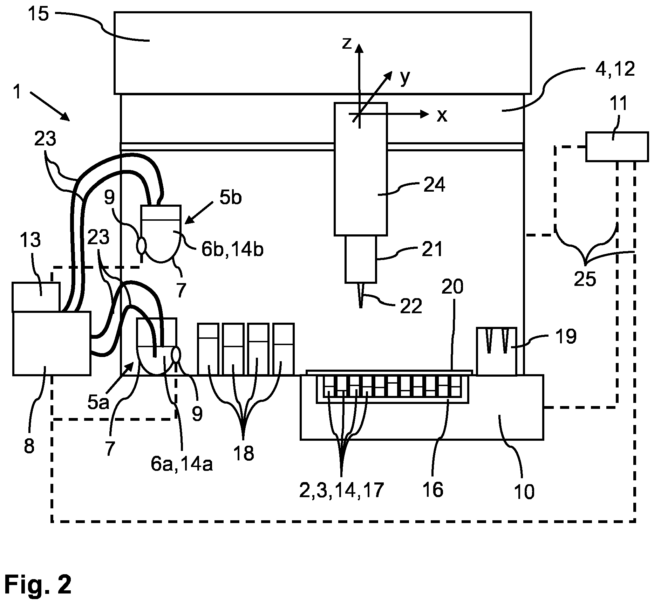

Die Figur 2 zeigt ein weiteres mögliches Ausgestaltungsbeispiel einer erfindungsgemäßen Apparatur 1 mit der, wie bereits bei dem Beispiel der Figur 1, ein erfindungsgemäßes Verfahren computer-implementiert ausgeführt werden kann. Im Unterschied zu der Apparatur 1 gemäß Figur 1 weist diejenige der Figur 2 einen zweiten Inokulum-Bioreaktor 5b auf. Während also in dem ersten Inokulum-Bioreaktionsgefäß 7a des ersten Inokulum-Bioreaktors 5a ein erster Organismus 14a bereitgehalten wird, stellt das zweite Inokulum 6b in dem zweiten Inokulum-Bioreaktionsgefäß 7b einen zweiten Organismus 14b bereit. Beide Inokulum-Bioreaktoren 5a und 5b werden dabei durch den gemeinsamen Regler 13 überwacht und geregelt. Mit dem Pipettier-Roboter 12 können so, insbesondere bei zunächst unveränderter Zusammensetzung des Kulturmediums 3, im jeweiligen Reaktionsgefäß 2 der Mikrotiterplatte 16 unterschiedliche Mengenverhältnisse zwischen den beiden Organismen 14a und 14b hergestellt werden, wobei die beiden Organismen 14a und 14b jeweils gemeinsam in dem Kulturmedium 3 im jeweiligen Reaktionsgefäß 2 kultiviert werden.

Auch bei der Ausgestaltung der Figur 2 können somit N Kultivierungsversuche jeweils parallel und gleichzeitig mit der Apparatur 1 durchgeführt werden, allerdings muss hier nicht zwingend die jeweilige Zusammensetzung des Kulturmediums 3 im jeweiligen Reaktionsgefäß 2 geändert werden, sondern der Controller 11 kann beispielsweise zunächst versuchen, das besagte Verhältnis der beiden Organismen 14a und 14b zu optimieren, etwa im Hinblick auf einen gewünschten Prozesszustand. Ein solcher Prozesszustand kann beispielsweise darin bestehen, dass die beiden Organismen 14a und 14b möglichst lange zusammen kultiviert werden können. Hat der Controller 11 über mehrere Versuchsreihen ein optimales Verhältnis zwischen den beiden Organismen 14a und 14b gefunden, so kann er anschließend noch zusätzlich selbständig erlernen, wie möglicherweise die Zusammensetzung des Kulturmediums 3 für das gefundene Verhältnis weiter optimiert werden kann. Selbstverständlich wären aber auch mit der Apparatur 1 der Figur 2 andere Optimierungsansätze möglich: Beispielsweise könnte der Controller 11 zunächst versuchen, eine Zusammensetzung des Kulturmediums 3 für einen der beiden Organismen 14a und 14b zu optimieren, um anschließend zu versuchen, in dieser gefundenen optimierten Zusammensetzung beide der Organismen 14a und 14b in einem geeigneten Mischungs-Verhältnis zu kultivieren.

Zusammenfassend wird zur Beschleunigung der Ermittlung optimaler Bedingungen zur Kultivierung eines bestimmten biologischen Organismus 14 eine Apparatur 1 und ein zugehöriges Verfahren vorgeschlagen, mit dem sich, mithilfe eines Computers und autonom, also ohne jegliches menschliches Zutun, eine optimierte Zusammensetzung eines Kulturmediums 3 und gegebenenfalls zusätzliche optimierte Wirkfaktoren, insbesondere ein optimiertes Verhältnis von (wenigstens) zwei Organismen (14a, 14b), die gemeinsam kultiviert werden sollen, ermitteln lassen. Hierfür ist vorgesehen, dass der zu untersuchende biologische Organismus 14, der in Form eines Inokulums 6 in einem separaten Inokulum-Bioreaktor 5 über eine gesamte Versuchsdauer am Leben und damit für die Versuche bereitgehalten wird, mithilfe eines Roboters 4 in einzelne Reaktionsgefäße 2 eingebracht wird, in denen sich festgelegte spezifische Zusammensetzungen des Kulturmediums 3 befinden und/oder bereits ein bestimmter Anteil eines zweiten Organismus 14. Anschließend wird der Organismus 14 in dem jeweiligen Kulturmedium 3, also innerhalb des jeweiligen Reaktionsgefäßes 2, kultiviert und mithilfe einer Prozessmessvorrichtung 10 eine Prozessantwort oder ein Prozesszustand gemessen, die der biologische Organismus 14 erzeugt bzw. in dem er sich gerade befindet. Schließlich bewertet die Apparatur 1 anhand einer Zielvorgabe, ob die vorgegebenen Kultivierungsbedingungen in dem jeweiligen Reaktionsgefäß 2 zu einer gewünschten Prozessantwort/Prozesszustand geführt haben.

Je nach Ergebnis dieser insgesamt N Bewertungen, wobei die jeweilige Bewertung für jedes der N Reaktionsgefäße 2 computer-implementier durchgeführt wird, kann dann von einem Controller 11 der Apparatur 1 entschieden werden, wie die Zusammensetzung des Kulturmediums 3 und gegebenenfalls weitere Wirkparameter in einer nachfolgenden Versuchsreihe vorgegeben und eingestellt oder verändert werden sollen. Hierzu werden dann in dieser nachfolgenden Versuchsreihe sämtliche Reaktionsgefäße 2 neu mit dem Kulturmedium 3 befüllt und auch neu mit dem zu untersuchenden Organismus 14 / den zu kultivierenden Organismen 14a, 14b automatisiert beimpft. Durch diesen Ansatz lässt sich in sehr kurzer Zeit die optimierte Zusammensetzung des Kulturmediums 3 für einen spezifischen biologischen Organismus 14 und/oder eine optimiertes Verhältnis von wenigstens zwei gemeinsam zu kultivierenden Organismen 14a, 14 automatisiert ermitteln.

### Bezugszeichenliste

- 1: Apparatur
- 2: Reaktionsgefäß
- 3: Kulturmedium
- 4: Roboter
- 5: Inokulum-Bioreaktor (kann 6 und 7 umfassen)
- 6: Inokulum (bereit gehalten in 7)
- 7: Inokulum-Reaktionsgefäß
- 8: Inokulum-Einstellmittel (zum Einstellen einer Zusammensetzung eines Kulturmediums von 6)
- 9: Inokulum-Messvorrichtung
- 10: Prozessmessvorrichtung
- 11: Controller
- 12: Pipettier-Roboter
- 13: Regler (zum Regeln der Brutbedingungen in 5)
- 14: Organismus
- 15: Filter-Modul (z.B. HEPA-Modul; zur Vermeidung von Kontaminationen)
- 16: Mikrotiterplatte
- 17: Mikrotiter-Kavität
- 18: Vorratsgefäß (zum Aufbewahren einer Medienkomponente)
- 19: Reinigungsstation für Pipettenspitze
- 20: Sterilbarriere
- 21: Pipette
- 22: Pipettenspitze
- 23: Schlauch
- 24: Pipettier-Arm (beweglich, zum Bewegen von 21/22)
- 25: Steuerleitungen (von 11, zum Auslesen und/oder Ansteuern von 4/12/10/8)

## Patentansprüche

1. **Apparatur (1) zur computer-implementierten Ermittlung einer optimierten Zusammensetzung eines Kulturmediums (3) und/oder eines optimierten Verhältnisses von wenigstens zwei Organismen (14a, 14b),** wobei die Apparatur (1),
- eine Anzahl an N räumlich getrennten Reaktionsgefäßen (2) aufweist, die mit N Zusammensetzungen des Kulturmediums (3) befüllbar sind, um darin, im Rahmen einer Versuchsreihe aus N Kultivierungsversuchen, jeweils mindestens einen biologischen Organismus (14) zu kultivieren, der einen biologischen Prozess ausführt,
- einen Roboter (4), der zum Herstellen der jeweiligen Zusammensetzung des Kulturmediums (3) in dem jeweiligen Reaktionsgefäß (2) eingerichtet ist, sowie
- wenigstens eine Prozessmessvorrichtung (10), die zum Messen einer jeweiligen Prozessantwort und/oder eines Prozesszustands eingerichtet ist, die/der innerhalb des jeweiligen Reaktionsgefäßes (2) von dem mindestens einen Organismus (14) während der Versuchsreihe erzeugt wird,
- vorzugsweise und einen separaten Inokulum-Bioreaktor (5), in welchem ein Inokulum (6) des zu kultivierenden Organismus (14) bereithaltbar oder bereitgehalten ist, **dadurch gekennzeichnet,**
- **dass** die Apparatur (1) einen Controller (11) aufweist, der dazu eingerichtet ist,
- die mittels der wenigstens einen Prozessmessvorrichtung (10) während der jeweiligen Versuchsreihe gemessenen insgesamt N Prozessantworten und/oder N Prozesszustände jeweils computer-implementiert anhand einer gemeinsamen Zielvorgabe, die für alle in den Reaktionsgefäßen (2) parallel, vorzugsweise gleichzeitig, ablaufenden Prozesse gilt, zu bewerten und auf Basis dieser N Bewertungen computer-implementiert
- die jeweilige Zusammensetzung des Kulturmediums (3) und/oder
- ein Verhältnis von wenigstens zwei unterschiedlichen Organismen (14a, 14b)
welche/welches in dem jeweiligen Reaktionsgefäß (2) in einer nachfolgenden Versuchsreihe hergestellt werden soll, dem Roboter (4) in Form einer Instruktion vorzugeben.

2. Apparatur (1) nach Anspruch 1, wobei der Roboter (4) als ein Pipettier-Roboter (12) ausgestaltet ist,
- vorzugsweise mit einem beweglichen Pipettier-Arm (24), der dazu eingerichtet ist, über dem jeweiligen der N Reaktionsgefäße (2) positioniert zu werden,
- insbesondere um so einzelne Komponenten des Kulturmediums (3) in das jeweilige Reaktionsgefäß (2) zu dispensieren und/oder um so das jeweilige Kulturmedium (3) aus dem jeweiligen Reaktionsgefäß (3) abzusaugen.

3. Apparatur (1) nach einem der vorhergehenden Ansprüche, wobei der Controller (11) den Roboter (4) mit Hilfe einer künstlichen Intelligenz steuert,
- insbesondere derart, dass die Apparatur (1) anhand der gemessenen Prozessantworten und/oder Prozesszustände im Laufe mehrerer zeitlich aufeinander folgender Versuchsreihen selbständig erlernt, wie der Roboter (4) eine optimierte Zusammensetzung des Kulturmediums (3) herstellen kann.

4. Apparatur (1) nach einem der vorhergehenden Ansprüche, wobei der Controller (11) über eine künstliche Intelligenz verfügt, insbesondere auf Basis maschinellen Lernens, die aus den jeweils gemessenen Prozessantworten selbständig, insbesondere ohne menschliches Eingreifen, eine optimierte Zusammensetzung des Kulturmediums (3) erlernt und diese, insbesondere am Ende einer mit der Apparatur (1) durchgeführten Sequenz von mehreren Versuchsreihen, ausgibt.

5. Apparatur (1) nach einem der vorhergehenden Ansprüche, wobei die Apparatur (1) wenigstens einen Inokulum-Bioreaktor (5) umfasst, in welchem ein Inokulum des zu kultivierenden Organismus (14) bereithaltbar oder bereitgehalten ist,
- insbesondere wobei der Roboter (4) dazu eingerichtet ist, eine Probe des Inokulums aus dem Inokulum-Bioreaktor (5) zu entnehmen und das jeweilige Reaktionsgefäß (2) mit dieser Probe zu beimpfen.

6. Apparatur (1) nach dem vorhergehenden Anspruch, wobei der Inokulum-Bioreaktor (5) umfasst:
- wenigstens ein, vorzugsweise temperaturgeregeltes, Inokulum-Reaktionsgefäß (7), in welchem das Inokulum (6) über mehrere Tage, vorzugsweise über wenigstens zwei Wochen, vorhaltbar ist und/oder
- wenigstens ein Inokulum-Einstellmittel (8) zum Anpassen einer Zusammensetzung des Inokulums (6), insbesondere
- wenigstens eine Pumpe zum Befördern eines Bestandteils eines Kulturmediums des Inokulums (6) in das Inokulum-Reaktionsgefäß (7) hinein und/oder
- wenigstens eine Pumpe zum Befördern eines Teils des Inokulums (6) aus dem Inokulum-Reaktionsgefäß (7) heraus,
und/oder
- wenigstens eine, vorzugsweise optische, Inokulum-Messvorrichtung (9), die zum Messen einer Volumenkonzentration des zu kultivierenden Organismus (14) innerhalb des Inokulums (6) eingerichtet ist.

7. Apparatur (1) nach dem vorhergehenden Anspruch,
- wobei ein Regler (13) des Inokulum-Bioreaktors (5) dazu eingerichtet ist, das wenigstens eine Inokulum-Einstellmittel (8) in Abhängigkeit einer mit der wenigstens einen Inokulum-Messvorrichtung (9) durchgeführten Messung so anzusteuern, dass eine Volumenkonzentration des zu kultivierenden Organismus (14) innerhalb des Inokulums (6) innerhalb eines vorgegebenen Intervalls gehalten wird.

8. Apparatur (1) nach einem der vorhergehenden Ansprüche,
- wobei der Roboter (4) dazu eingerichtet ist,
- insbesondere nach Durchführung einer Versuchsreihe, in welcher der Organismus (14) in unterschiedlichen Zusammensetzungen des Kulturmediums (3) in den einzelnen Reaktionsgefäßen (2) kultiviert wurde,
die Reaktionsgefäße (2) zu reinigen,
- vorzugsweise durch Ausspülen und/oder durch Desinfizieren, insbesondere durch Spülen mit einem Desinfektionsmittel,
- besonders bevorzugt wobei der Roboter (3) beim Reinigen und Desinfizieren der Reaktionsgefäße (2) einem festgelegten Ablaufplan folgt, der mehrere Spülschritte und ein Warteintervall umfasst, in welchem das Desinfektionsmittel auf das jeweilige Reaktionsgefäß (2) einwirkt.

9. Apparatur (1) nach einem der vorhergehenden Ansprüche,
- wobei die wenigstens eine Prozessmessvorrichtung (10) eine optische Prozessmessvorrichtung (10) ist,
- vorzugsweise wobei die optische Prozessmessvorrichtung (10) eine optische Transmissionsmessung und/oder eine optische Rückstreumessung realisiert.

10. Apparatur (1) nach einem der vorhergehenden Ansprüche,
- wobei die Apparatur (1) nur über eine Prozessmessvorrichtung (10) verfügt, mit der jedes der Reaktionsgefäße (2), insbesondere sequentiell nacheinander, vermessen werden kann, oder
- wobei jedes der Reaktionsgefäße (2) über eine eigene Prozessmessvorrichtung (10) verfügt, mit der eine jeweilige Prozessantwort, die der Organismus in dem jeweiligen Reaktionsgefäß (2) erzeugt, insbesondere gleichzeitig, messbar ist und/oder
- wobei die Apparatur (1) zur Ausführung eines Verfahrens gemäß einem der nachfolgenden Ansprüche eingerichtet ist.

11. **Verfahren zur computer-implementierten Ermittlung einer optimierten Zusammensetzung eines Kulturmediums (3),** in welchem wenigstens ein Organismus (14) kultiviert werden soll, **und/oder eines optimierten Verhältnisses von wenigstens zwei Organismen (14a, 14b),** die gemeinsam in einem Kulturmedium kultiviert werden sollen,
- insbesondere wobei das Verfahren mittels einer Apparatur (1) nach einem der vorhergehenden Ansprüche implementiert wird, **dadurch gekennzeichnet,**
- **dass** eine Anzahl an N räumlich getrennten Reaktionsgefäßen (2) jeweils mit einer festgelegten Zusammensetzung des Kulturmediums (3), vorzugsweise mit Hilfe eines Roboters (4) automatisiert, befüllt werden,
- **dass** die N Reaktionsgefäße (2), vorzugsweise mit dem Roboter (4), mit einem Inokulum (6), welches den jeweiligen Organismus (14) umfasst, beimpft werden,
- **dass** nachfolgend eine Versuchsreihe aus N Kultivierungsversuchen, die parallel, vorzugsweise gleichzeitig, in den N Reaktionsgefäßen (2) mit dem Organismus (14)/den wenigstens zwei Organismen (14a, 14b) ablaufen, durchgeführt wird, wobei der jeweilige Organismus (14) in jedem der N Reaktionsgefäße (2) einen jeweiligen biologischen Prozess ausführt,
- **dass**, insbesondere während und/oder am Ende der Versuchsreihe, eine jeweilige Prozessantwort und/oder ein jeweiliger Prozesszustand, die/den der Organismus (14) in dem jeweiligen Reaktionsgefäß (2) erzeugt, mit wenigstens einer Prozessmessvorrichtung (10) gemessen wird,
- **dass** die N gemessenen Prozessantworten und/oder Prozesszustände computer-implementiert, vorzugsweise unter Einsatz einer künstlichen Intelligenz, jeweils anhand einer Zielvorgabe bewertet werden, und
- **dass** auf Basis dieser N Bewertungen computer-implementiert
- eine jeweilige Zusammensetzung des Kulturmediums (3) und/oder
- ein Verhältnis der wenigstens zwei unterschiedlichen Organismen (14),
die/das in dem jeweiligen Reaktionsgefäß (2) in einer nachfolgenden Versuchsreihe angelegt und/oder die/das während eines nachfolgenden jeweiligen Kultivierungsversuchs verändert werden soll, mittels einer Instruktion computer-implementiert vorgegeben wird.

12. Verfahren gemäß dem vorherigen Anspruch, wobei der Roboter zu Beginn der nachfolgenden Versuchsreihe die Instruktion ausführt, indem er in jedem der N Reaktionsgefäße (2) die jeweils durch die Instruktion vorgegebene Zusammensetzung des Kulturmediums (3) und/oder das jeweils durch die Instruktion vorgegebene Verhältnis der wenigstens zwei Organismen herstellt und/oder
- wobei während eines laufenden Kultivierungsversuchs die jeweilige Zusammensetzung des Kulturmediums und/oder das jeweilige Verhältnis der wenigstens zwei unterschiedlichen Organismen (14) in dem jeweiligen Reaktionsgefäß verändert wird/werden, insbesondere mit Hilfe des Roboters (4).

13. Verfahren gemäß Anspruch 11 oder 12, wobei die Versuchsreihen iterativ als eine Sequenz aus mehreren zeitlich aufeinander folgenden Versuchsreihen durchgeführt werden,
- vorzugsweise wobei schrittweise die Zusammensetzung des Kulturmediums (3) und/oder das besagte Verhältnis auf Basis der jeweils in den einzelnen Versuchsreihen bestimmten N Bewertungen an ein Optimum herangeführt wird,
- insbesondere sodass wenigstens eine der N gemessenen Prozessantworten näher an die Zielvorgabe heranrückt.

14. Verfahren gemäß einem der Ansprüche 11 bis 13, wobei die Reaktionsgefäße (2) am Ende der jeweiligen Versuchsreihe, vorzugsweise mit Hilfe des Roboters (4), gereinigt und/oder desinfiziert werden, insbesondere durch Ausspülen des Kulturmediums (3) und nachfolgender Behandlung des jeweiligen Reaktionsgefäßes (2) mit einem Desinfektionsmittel.

15. Verfahren gemäß einem der Ansprüche 11 bis 14, wobei das Inokulum (6), mit welchem die N Reaktionsgefäße (2) zu Beginn einer jeden Versuchsreiche neu beimpft werden, aus einem separaten Inokulum-Bioreaktor (5), vorzugsweise mit Hilfe des Roboters (4), entnommen werden,
- vorzugsweise wobei Brutbedingungen des Inokulums (6) und/oder eine Volumenkonzentration des Organismus (14) in dem Inokulum (4) automatisiert durch den Inokulum-Bioreaktor (5) während einer gesamten Dauer einer Sequenz der Versuchsreihen konstant gehalten werden.
